# EUROPEAN PATENT APPLICATION

(11) **EP 4 438 606 A1**
(43) Date of publication of application: **02.10.2024**
(21) Application number: 22909132.7
(22) Date of filing: 17.05.2022
(51) Int. Cl.: C07D 487/04, A61K 31/53, A61P 31/12, A61P 31/14

(54) **NEW CRYSTAL FORM OF NUCLEOSIDE COMPOUND AND SALT THEREOF**

(30) Priority: 23.12.2021 CN 202111593324
(71) Applicant: Shenzhen Antiv Pharma Co., Ltd., Shenzhen, Guangdong province 518118 (CN)
(72) Inventor: LI, Shuo, Shenzhen, Guangdong province (CN); LI, Guanguan, Shenzhen, Guangdong province (CN); LIU, Xinjun, Shenzhen, Guangdong province (CN); LI, Yingjun, Shenzhen, Guangdong province (CN); ZHOU, Qifan, Shenzhen, Guangdong province (CN)
(74) Representative: Laufhütte, Dieter
(86) International application number: PCT/CN2022/093342
(87) International publication number: WO 2023/115796

(57) **Abstract**

The present invention provides novel crystalline forms of a nucleoside compound and salts thereof, which belongs to the technical field of medicines. The nucleoside compound is compound ATV014. The present invention focuses on investigating whether compound ATV014 has multiple solid forms. Surprisingly, it is discovered that compound ATV014 exists in a variety of stable solid forms, comprising: ATV-014 free base crystalline form I, ATV-014 hydrochloride crystalline form I, ATV-014 hydrochloride crystalline form II, ATV-014 hydrochloride crystalline form III, ATV-014 hydrochloride crystalline form IV, ATV-014 hydrobromide crystalline form I, and ATV-014 *p*-toluenesulfonate crystalline form I. The provided novel crystalline forms of the compound ATV014 free base and salts thereof have good solubility and thermal stability, and better bioavailability and dissolution curve in a specific preparation; and which are suitable for use in a specific pharmaceutical preparation due to the good permeability and thermal stability thereof.

## Description

### TECHNICAL FIELD

The present invention belongs to the technical field of medicines, and provides novel crystalline forms of a nucleoside compound and a salt thereof.

### BACKGROUND

Coronaviruses is an enveloped, single-stranded RNA viruse, belonging to the genus β coronavirus. Similar to SARS and MERS, the SARS-CoV-2 genome encodes non-structural proteins including 3-chymotrypsin-like protease (3CLPro), papain-like protease (PLPro), helicase and RNA-dependent RNA polymerase (RdRp), as well as structural proteins such as spike glycoproteins and accessory proteins. The surface spike protein of SARS-CoV-2 binds the angiotensin converting enzyme 2 (ACE2) receptor on the surface of human cells to infect human respiratory epithelial cells. After entering the host cell, the virus disintegrates and the nucleocapsid and viral RNA was released into the cytoplasm. The open reading frame at the 5' -terminal of viral RNA (ORF1ab) encodes polyproteins (pp1a and pplab), which play an essential role in the processing and maturation of enzymes required for viral replication. PPla and PPlab can be cleaved by PLPro and 3CLPro to produce non-structural proteins, including helicase and RNA-dependent RNA polymerase etc, which play a key role in the transcription and replication of SARS-CoV-2. Currently, these four proteins, including the surface spike glycoprotein of coronavirus recognition receptor, 3CLpro, PLpro and RdRp, that are important proteins involved in viral reproduction and transcription process, are very attractive targets for the development of antiviral drugs.

Through previous studies by the applicant on Remdesivir and its prodrug GS-441524 (Li, et al., J. Med. Chem. 2020), it was found that GS-441524 produced an antiviral effect superior to that of Remdesivir in vivo in mice. GS-441524 shows better safety although its mechanism of action is similar to that of Remdesivir. Therefore, the applicant has filed a patent application (application number or patent number 202011000517.2) describing the use of GS-441524 in the prevention, lessening, and/or treatment of SARS-CoV-2.

The structure of GS-441524 is as follows:

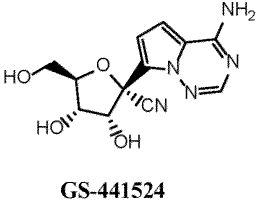

The compound ATV014 is a derivative of GS-441524. The inventor has found that the compound ATV014 has better activity and bioavailability compared to GS-441524.

The structure of ATV014 is as follows:

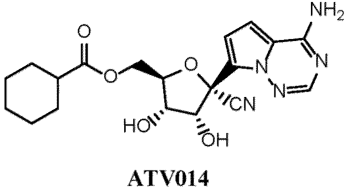

Drug polymorphism is a common phenomenon in drug research and an important factor affecting drug quality. Different crystalline forms of the same drug may have significant differences in physical and chemical properties such as appearance, fluidity, solubility, storage stability, and bioavailability, which may have significant impacts on the storage, transfer, application, stability, and efficacy of the drug; In order to obtain effective crystalline forms that are beneficial for production or drug formulations, it is necessary to comprehensively investigate the crystallization behavior of drugs to obtain crystalline forms that meet production requirements.

The present invention has obtained novel crystalline forms of compound ATV014 and its salt through many experimental studies. The novel crystalline forms have superior properties such as good stability, stability under high temperature and humidity conditions under light, simple preparation process and easy operation, and has advantages in industrial production. The studies of the novel crystalline forms of compound ATV014 provide an opportunity to improve the overall performance of the pharmaceutical product (such as easy to synthesis or processing, increased solubility, or increased stability and shelf life), while expanding the variety of materials available for formulation scientists to design the drug, which is crucial for drug development.

### SUMMARY

The present disclosure aims to solve one of the technical problems in related art at least to some extent. For this purpose, one objective of the present invention is to provide a novel crystalline form and preparation method for compound ATV014 free base and its salts. The present invention focuses on investigating whether compound ATV014 has multiple solid forms. Surprisingly, it is discovered that compound ATV014 exists in a variety of stable solid forms, comprising: ATV-014 free base crystalline form I, ATV-014 hydrochloride crystalline form I, ATV-014 hydrochloride crystalline form II, ATV-014 hydrochloride crystalline form III, ATV-014 hydrochloride crystalline form IV, ATV-014 hydrobromide crystalline form I, and ATV-014 *p*-toluenesulfonate crystalline form I.

Wherein the structure of ATV014 is as follows:

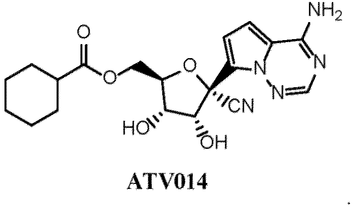

Novel crystalline forms of the compound ATV014 free base and the salts thereof provided herein have good solubility and thermal stability, and better bioavailability and dissolution curve in a specific preparation; and which are suitable for use in a specific pharmaceutical preparation due to the good permeability and thermal stability thereof.

### DETAILED DESCRIPTION OF THE INVENTION

To solve the problems above, the present disclosure provides the following technical solution:
In a first aspect, there is provided a crystalline form of compound ATV-014 free base or its salts.

According to one aspect, the present invention provided ATV-014 free base crystalline form I, ATV-014 hydrochloride crystalline form I, ATV-014 hydrochloride crystalline form II, ATV-014 hydrochloride crystalline form III, ATV-014 hydrochloride crystalline form IV, ATV-014 hydrobromide crystalline form I, and ATV-014 *p*-toluenesulfonate crystalline form I.

### Compound ATV-014 free base crystalline form I

In some embodiments, there is provided compound ATV-014 free base crystalline form I, wherein the free base crystalline form I is an anhydrous substance.

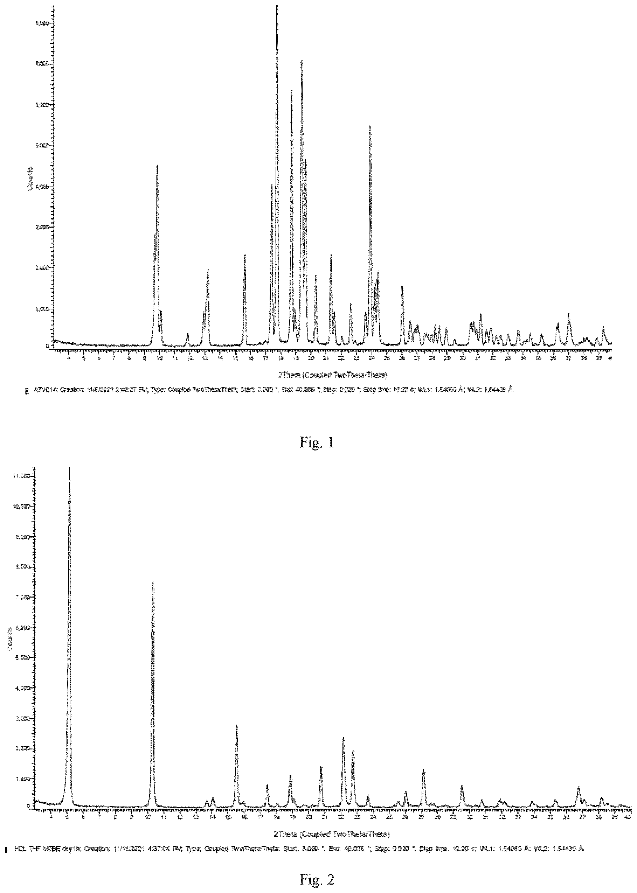

In some embodiments, compound ATV-014 free base crystalline form I has an X-ray powder diffraction pattern (XRPD pattern) substantially as the one shown in FIG. 1.

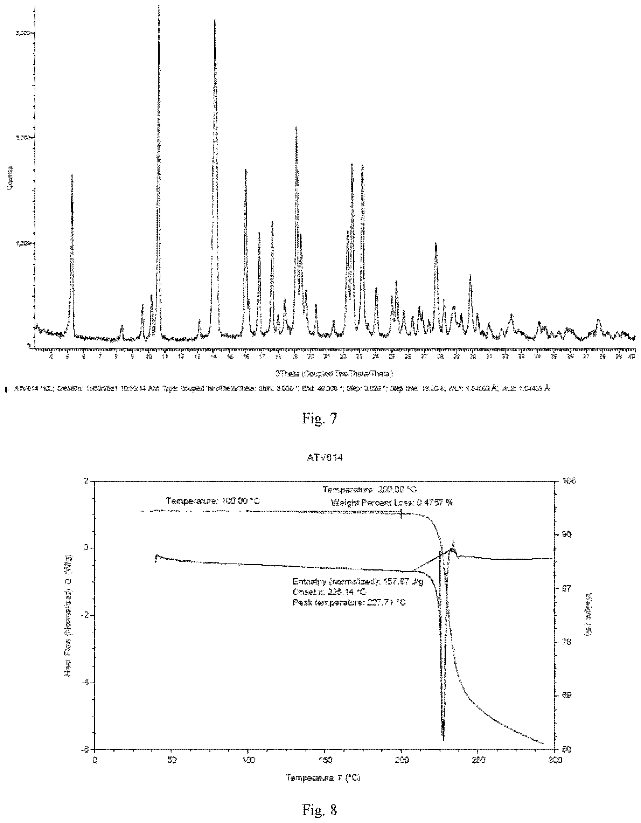

In some embodiments, compound ATV-014 free base crystalline form I has a differential scanning calorimetry curve (DSC) substantially as the one shown in FIG. 8.

In some embodiments, compound ATV-014 free base crystalline form I has a thermogravimetric analysis curve (TGA) substantially as the one shown in FIG. 8.

In some embodiments, compound ATV-014 free base crystalline form I is applicable to at least one, at least two, or all of the following (a) - (c): (a) crystalline form I has an X-ray powder diffraction pattern (XRPD pattern) substantially as depicted in FIG. 1; (b) crystalline form I has a differential scanning calorimetry curve (DSC) substantially as depicted in FIG. 8; (c) crystalline form I has a thermogravimetric analysis curve (TGA) substantially as depicted in FIG. 8.

In some embodiments, compound ATV-014 free base crystalline form I has the following properties:
(a) crystalline form I has an X-ray powder diffraction pattern (XRPD pattern) substantially as depicted in FIG. 1;
(b) crystalline form I has a differential scanning calorimetry curve (DSC) substantially as depicted in FIG. 8;
(c) crystalline form I has a thermogravimetric analysis curve (TGA) substantially as depicted in FIG. 8.

In some embodiments, compound ATV-014 free base crystalline form I has at least 2, at least 3, at least 4, at least 5, or at least 6 XRPD patterns with 2 θ degree reflections, and its maximum intensity is substantially as depicted in FIG. 1.

In some embodiments, the differential scanning calorimetry curve of compound ATV-014 free base crystalline form I has an endothermic peak with a peak temperature of 225 °C-228 °C.

In some embodiments, the thermogravimetric analysis curve of compound ATV014 free base crystalline form I have a weight loss of below 0.5% in a temperature range of 100 °C to 200 °C.

In some embodiments, the X-ray powder diffraction pattern of compound ATV-014 free base crystalline form I has characteristic peaks at 2Θ ± 0.2° values of 9.69, 18.70 and 23.88.

In some embodiments, the X-ray powder diffraction pattern of compound ATV-014 free base crystalline form I has characteristic peaks at 2Θ ± 0.2° values of 9.69, 18.70 and 23.88, and further has one, two or three characteristic peaks at 2Θ ± 0.2° values of 9.84, 17.74 and 19.38.

In some embodiments, the X-ray powder diffraction pattern of compound ATV-014 free base crystalline form I has characteristic peaks at 2Θ ± 0.2° values of 9.69, 18.70 and 23.88, and further has one or two characteristic peaks at 2Θ ± 0.2° values of 9.84, 17.74 and 19.38.

In some embodiments, the X-ray powder diffraction pattern of compound ATV-014 free base crystalline form I has characteristic peaks at 2Θ ± 0.2° values of 9.69, 18.70 and 23.88, and further has one characteristic peak at 2Θ ± 0.2° values of 9.84, 17.74 and 19.38.

In some embodiments, the X-ray powder diffraction pattern of compound ATV-014 free base crystalline form I has characteristic peaks at 2Θ ± 0.2° values of 9.69, 18.70 and 23.88, and further has two characteristic peaks at 2Θ ± 0.2° values of 9.84, 17.74 and 19.38.

In some embodiments, the X-ray powder diffraction pattern of compound ATV-014 free base crystalline form I has characteristic peaks at 2Θ ± 0.2° values of 9.69, 9.84, 17.74, 18.70, 19.38 and 23.88.

In some embodiments, the X-ray powder diffraction pattern of compound ATV-014 free base crystalline form I has at least three characteristic peaks at 2Θ ± 0.2° values of 9.69, 9.84, 17.74, 18.70, 19.38 and 23.88.

In some embodiments, the X-ray powder diffraction pattern of compound ATV-014 free base crystalline form I has characteristic peaks at 2Θ ± 0.2° values of 9.69, 9.84, 17.74, 18.70, 19.38 and 23.88, and further has one, two or three characteristic peaks at 2Θ ± 0.2° values of 13.19, 15.61 and 21.31.

In some embodiments, the X-ray powder diffraction pattern of compound ATV-014 free base crystalline form I has characteristic peaks at 2Θ ± 0.2° values of 9.69, 9.84, 17.74, 18.70, 19.38 and 23.88, and further has one or two characteristic peaks at 2Θ ± 0.2° values of 13.19, 15.61 and 21.31.

In some embodiments, the X-ray powder diffraction pattern of compound ATV-014 free base crystalline form I has characteristic peaks at 2Θ ± 0.2° values of 9.69, 9.84, 17.74, 18.70, 19.38 and 23.88, and further has one characteristic peak at 2Θ ± 0.2° values of 13.19, 15.61 and 21.31.

In some embodiments, the X-ray powder diffraction pattern of compound ATV-014 free base crystalline form I has characteristic peaks at 2Θ ± 0.2° values of 9.69, 9.84, 17.74, 18.70, 19.38 and 23.88, and further has two characteristic peaks at 2Θ ± 0.2° values of 13.19, 15.61 and 21.31.

In some embodiments, the X-ray powder diffraction pattern of compound ATV-014 free base crystalline form I has characteristic peaks at 2Θ ± 0.2° values of 9.69, 9.84, 13.91, 15.61, 17.74, 18.70, 19.38, 21.31 and 23.88.

In some embodiments, the X-ray powder diffraction pattern of compound ATV-014 free base crystalline form I has at least three characteristic peaks at 2Θ ± 0.2° values of 9.69, 9.84, 13.91, 15.61, 17.74, 18.70, 19.38,21.31 and 23.88.

In some embodiments, the X-ray powder diffraction pattern of compound ATV-014 free base crystalline form I has characteristic peaks at 2Θ ± 0.2° values of 9.69, 9.84, 13.91, 15.61, 17.74, 18.70, 19.38, 21.31 and 23.88, and further has one or two characteristic peaks at 2Θ ± 0.2° values of 17.41 and 26.01.

In some embodiments, the X-ray powder diffraction pattern of compound ATV-014 free base crystalline form I has characteristic peaks at 2Θ ± 0.2° values of 9.69, 9.84, 13.91, 15.61, 17.74, 18.70, 19.38, 21.31 and 23.88, and further has one characteristic peak at 2Θ ± 0.2° values of 17.41 and 26.01.

In some embodiments, the X-ray powder diffraction pattern of compound ATV-014 free base crystalline form I has characteristic peaks at 2Θ ± 0.2° values of 9.69, 9.84, 13.91, 15.61, 17.41, 17.74, 18.70, 19.38, 21.31, 23.88 and 26.01.

In some embodiments, the X-ray powder diffraction pattern of compound ATV-014 free base crystalline form I has at least three characteristic peaks at 2Θ ± 0.2° values of 9.69, 9.84, 13.91, 15.61, 17.41, 17.74, 18.70, 19.38, 21.31, 23.88 and 26.01.

### Compound ATV-014 hydrochloride crystalline form I

In some embodiments, there is provided compound ATV-014 hydrochloride crystalline form I, wherein the hydrochloride crystalline form I is a solvate.

In some embodiments, the hydrochloride crystalline form I has an X-ray powder diffraction pattern (XRPD pattern) substantially as the one shown in FIG. 2.

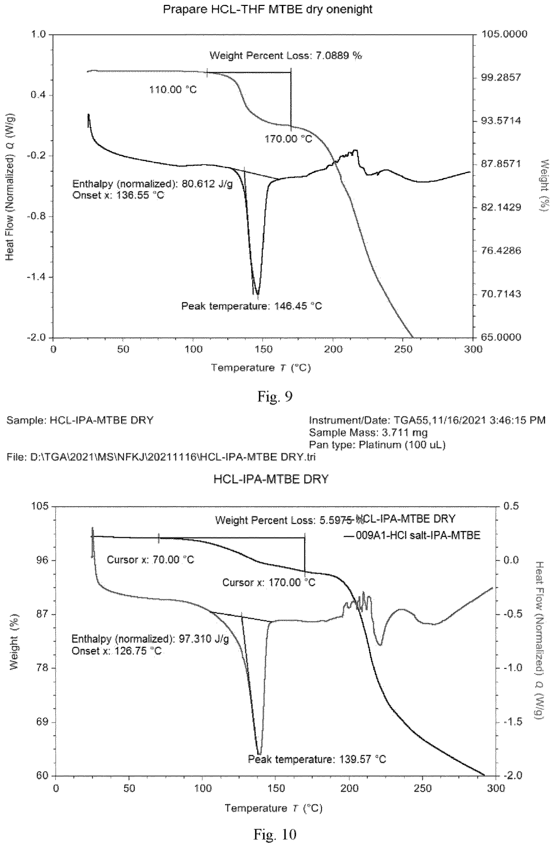

In some embodiments, the hydrochloride crystalline form I has a differential scanning calorimetry curve (DSC) substantially as the one shown in FIG. 9.

In some embodiments, the hydrochloride crystalline form I has a thermogravimetric analysis curve (TGA) substantially as the one shown in FIG. 9.

In some embodiments, compound ATV-014 hydrochloride crystalline form I is applicable to at least one, at least two, or all of the following (a) - (c): (a) crystalline form I has an X-ray powder diffraction pattern (XRPD pattern) substantially as depicted in FIG. 2; (b) crystalline form I has a differential scanning calorimetry curve (DSC) substantially as depicted in FIG. 9; (c) crystalline form I has a thermogravimetric analysis curve (TGA) substantially as depicted in FIG. 9.

In some embodiments, compound ATV-014 hydrochloride crystalline form I has the following properties:
(a) crystalline form I has an X-ray powder diffraction pattern (XRPD pattern) substantially as depicted in FIG. 2;
(b) crystalline form I has a differential scanning calorimetry curve (DSC) substantially as depicted in the differential scanning calorimetry curve in FIG. 9;
(c) crystalline form I has a thermogravimetric analysis curve (TGA) substantially as depicted in the thermogravimetric analysis curve in FIG. 9.

In some embodiments, compound ATV-014 hydrochloride crystalline form I has at least 2, at least 3, at least 4, at least 5, or at least 6 XRPD patterns with 2 θ degree reflections, and its maximum intensity is substantially as depicted in FIG.2.

In some embodiments, the differential scanning calorimetry curve of compound ATV-014 hydrochloride crystalline form I has an endothermic peak with a peak temperature of 120 °C-170 °C. In some embodiments, the differential scanning calorimetry curve of compound ATV-014 hydrochloride crystalline form I has an endothermic peak with a peak temperature of 125 °C - 165 °C. In some embodiments, the differential scanning calorimetry curve of compound ATV-014 hydrochloride crystalline form I has an endothermic peak with a peak temperature of 141°C - 151 °C or 143 °C - 149 °C. In some embodiments, compound ATV-014 hydrochloride crystalline form I has a differential scanning calorimetry curve substantially as the one shown in FIG. 9.

In some embodiments, the differential scanning calorimetry curve of compound ATV-014 hydrochloride crystalline form I shows its melting point as 136.55±5°C.

In some embodiments, the differential scanning calorimetry curve of compound ATV-014 hydrochloride crystalline form I has an endothermic peak with a peak temperature of 137 °C - 146 °C, the enthalpy value is 81 J/g, which represents the melting peak of desolvent merging.

In some embodiments, the thermogravimetric analysis curve of compound ATV014 hydrochloride crystalline form I has a weight loss of less than 10% or less than 9% or less than 8% in a temperature range of 110 °C to 170 °C. In some embodiments, the thermogravimetric analysis curve of compound ATV014 hydrochloride crystalline form I has a weight loss of below 7.1% in a temperature range of 110 °C to 170 °C.

In some embodiments, the X-ray powder diffraction pattern of compound ATV-014 hydrochloride crystalline form I has characteristic peaks at 2Θ ± 0.2° values of 5.16, 15.53 and 20.76.

In some embodiments, the X-ray powder diffraction pattern of compound ATV-014 hydrochloride crystalline form I has characteristic peaks at 2Θ ± 0.2° values of 5.16, 15.53 and 20.76, and further has one, two or three characteristic peaks at 2Θ ± 0.2° values of 10.33, 22.75 and 24.19.

In some embodiments, the X-ray powder diffraction pattern of compound ATV-014 hydrochloride crystalline form I has characteristic peaks at 2Θ ± 0.2° values of 5.16, 15.53 and 20.76, and further has one or two characteristic peaks at 2Θ ± 0.2° values of 10.33, 22.75 and 24.19.

In some embodiments, the X-ray powder diffraction pattern of compound ATV-014 hydrochloride crystalline form I has characteristic peaks at 2Θ ± 0.2° values of 5.16, 15.53 and 20.76, and further has one characteristic peak at 2Θ ± 0.2° values of 10.33, 22.75 and 24.19.

In some embodiments, the X-ray powder diffraction pattern of compound ATV-014 hydrochloride crystalline form I has characteristic peaks at 2Θ ± 0.2° values of 5.16, 15.53 and 20.76, and further has two characteristic peaks at 2Θ ± 0.2° values of 10.33, 22.75 and 24.19.

In some embodiments, the X-ray powder diffraction pattern of compound ATV-014 hydrochloride crystalline form I has characteristic peaks at 2Θ ± 0.2° values of 5.16, 10.33, 15.53, 20.76, 22.75 and 24.19.

In some embodiments, the X-ray powder diffraction pattern of compound ATV-014 hydrochloride crystalline form I has at least three characteristic peaks at 2Θ ± 0.2° values of 5.16, 10.33, 15.53, 20.76, 22.75 and 24.19.

In some embodiments, the X-ray powder diffraction pattern of compound ATV-014 hydrochloride crystalline form I has characteristic peaks at 2Θ ± 0.2° values of 5.16, 10.33, 15.53, 20.76, 22.75 and 24.19, and further has one or two characteristic peaks at 2Θ ± 0.2° values of 23.88 and 26.01.

In some embodiments, the X-ray powder diffraction pattern of compound ATV-014 hydrochloride crystalline form I has characteristic peaks at 2Θ ± 0.2° values of 5.16, 10.33, 15.53, 20.76, 22.75 and 24.19, and further has one characteristic peak at 2Θ ± 0.2° values of 23.88 and 26.01.

In some embodiments, the X-ray powder diffraction pattern of compound ATV-014 hydrochloride crystalline form I has characteristic peaks at 2Θ ± 0.2° values of 5.16, 10.33, 15.53, 20.76, 22.75, 23.88, 24.19 and 26.01.

In some embodiments, the X-ray powder diffraction pattern of compound ATV-014 hydrochloride crystalline form I has at least three characteristic peaks at 2Θ ± 0.2° values of 5.16, 10.33, 15.53, 20.76, 22.75, 23.88, 24.19 and 26.01.

### Compound ATV-014 hydrochloride crystalline form II

In some embodiments, there is provided compound ATV-014 hydrochloride crystalline form II, wherein the hydrochloride crystalline form II is a solvate/hydrate.

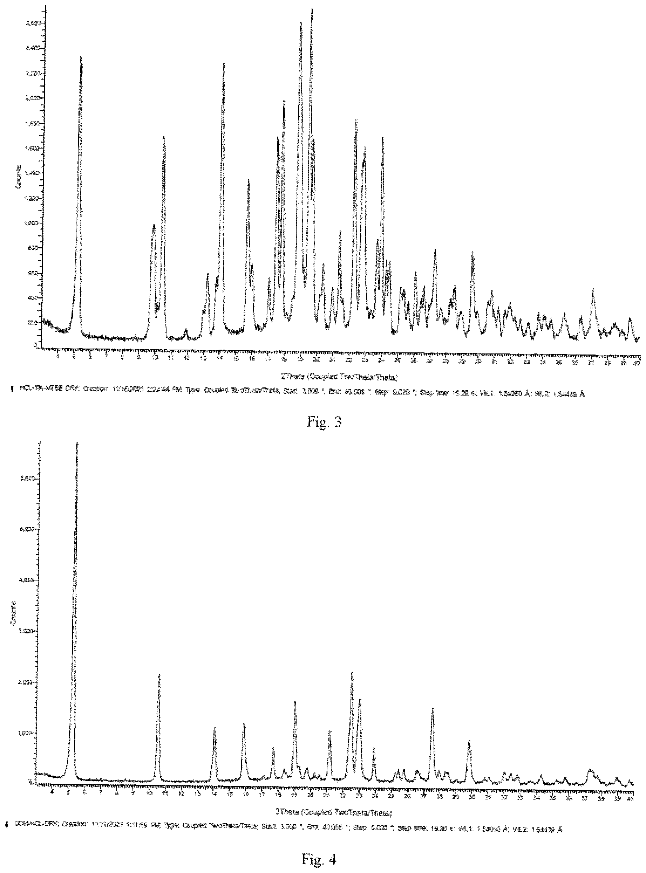

In some embodiments, the hydrochloride crystalline form II has an X-ray powder diffraction pattern (XRPD pattern) substantially as the one shown in FIG. 3.

In some embodiments, the hydrochloride crystalline form II has a differential scanning calorimetry curve (DSC) substantially as the one shown in FIG. 10.

In some embodiments, the hydrochloride crystalline form II has a thermogravimetric analysis curve (TGA) substantially as the one shown in FIG. 10.

In some embodiments, compound ATV-014 hydrochloride crystalline form II is applicable to at least one, at least two, or all of the following (a) - (c): (a) crystalline form II has an X-ray powder diffraction pattern (XRPD pattern) substantially as depicted in FIG. 3; (b) crystalline form II has a differential scanning calorimetry curve (DSC) substantially as depicted in FIG. 10; (c) crystalline form II has a thermogravimetric analysis curve (TGA) substantially as depicted in FIG. 10.

In some embodiments, compound ATV-014 hydrochloride crystalline form II has the following properties:
(a) crystalline form II has an X-ray powder diffraction pattern (XRPD pattern) substantially as depicted in FIG. 3;
(b) crystalline form II has a differential scanning calorimetry curve (DSC) substantially as depicted in the differential scanning calorimetry curve in FIG. 10;
(c) crystalline form II has a thermogravimetric analysis curve (TGA) substantially as depicted in the thermogravimetric analysis curve in FIG. 10.

In some embodiments, compound ATV-014 hydrochloride crystalline form II has at least 2, at least 3, at least 4, at least 5, or at least 6 XRPD patterns with 2 θ degree reflections, and its maximum intensity is substantially as depicted in FIG.3.

In some embodiments, the differential scanning calorimetry curve of compound ATV-014 hydrochloride crystalline form II has an endothermic peak with a peak temperature of 100 °C- 150 °C. In some embodiments, the differential scanning calorimetry curve of compound ATV-014 hydrochloride crystalline form II has an endothermic peak with a peak temperature of 135 °C - 145 °C. In some embodiments, the differential scanning calorimetry curve of compound ATV-014 hydrochloride crystalline form II has an endothermic peak with a peak temperature of 136 °C - 143 °C. In some embodiments, compound ATV-014 hydrochloride crystalline form II has a differential scanning calorimetry curve substantially as the one shown in FIG. 10.

In some embodiments, the differential scanning calorimetry curve of compound ATV-014 hydrochloride crystalline form II shows its melting point as 126.75±5°C.

In some embodiments, the thermogravimetric analysis curve of compound ATV014 hydrochloride crystalline form II has a weight loss of less than 10% in a temperature range of 70 °C to 170 °C. In some embodiments, the thermogravimetric analysis curve of compound ATV014 hydrochloride crystalline form II has a weight loss of less than 8% in a temperature range of 70 °C to 170 °C. In some embodiments, the thermogravimetric analysis curve of compound ATV014 hydrochloride crystalline form II has a weight loss of below 5.6% in a temperature range of 70 °C to 170 °C.

In some embodiments, the X-ray powder diffraction pattern of compound ATV-014 hydrochloride crystalline form II has characteristic peaks at 2Θ ± 0.2° values of 5.23, 10.43 and 18.79.

In some embodiments, the X-ray powder diffraction pattern of compound ATV-014 hydrochloride crystalline form II has characteristic peaks at 2Θ ± 0.2° values of 5.23, 10.43 and 18.79, and further has one, two or three characteristic peaks at 2Θ ± 0.2° values of 9.88, 14.05 and 22.28.

In some embodiments, the X-ray powder diffraction pattern of compound ATV-014 hydrochloride crystalline form II has characteristic peaks at 2Θ ± 0.2° values of 5.23, 10.43 and 18.79, and further has one or two characteristic peaks at 2Θ ± 0.2° values of 9.88, 14.05 and 22.28.

In some embodiments, the X-ray powder diffraction pattern of compound ATV-014 hydrochloride crystalline form II has characteristic peaks at 2Θ ± 0.2° values of 5.23, 10.43 and 18.79, and further has one characteristic peak at 2Θ ± 0.2° values of 9.88, 14.05 and 22.28.

In some embodiments, the X-ray powder diffraction pattern of compound ATV-014 hydrochloride crystalline form II has characteristic peaks at 2Θ ± 0.2° values of 5.23, 10.43 and 18.79, and further has two characteristic peaks at 2Θ ± 0.2° values of 9.88, 14.05 and 22.28.

In some embodiments, the X-ray powder diffraction pattern of compound ATV-014 hydrochloride crystalline form II has characteristic peaks at 2Θ ± 0.2° values of 5.23, 9.88, 10.43, 14.05, 18.79 and 22.28.

In some embodiments, the X-ray powder diffraction pattern of compound ATV-014 hydrochloride crystalline form II has at least three characteristic peaks at 2Θ ± 0.2° values of 5.23, 9.88, 10.43, 14.05, 18.79 and 22.28.

In some embodiments, the X-ray powder diffraction pattern of compound ATV-014 hydrochloride crystalline form II has characteristic peaks at 2Θ ± 0.2° values of 5.23, 9.88, 10.43, 14.05, 18.79 and 22.28, and further has one, two or three characteristic peaks at 2Θ ± 0.2° values of 15.68, 19.46 and 21.38.

In some embodiments, the X-ray powder diffraction pattern of compound ATV-014 hydrochloride crystalline form II has characteristic peaks at 2Θ ± 0.2° values of 5.23, 9.88, 10.43, 14.05, 18.79 and 22.28, and further has one or two characteristic peaks at 2Θ ± 0.2° values of 15.68, 19.46 and 21.38.

In some embodiments, the X-ray powder diffraction pattern of compound ATV-014 hydrochloride crystalline form II has characteristic peaks at 2Θ ± 0.2° values of 5.23, 9.88, 10.43, 14.05, 18.79 and 22.28, and further has one characteristic peak at 2Θ ± 0.2° values of 15.68, 19.46 and 21.38.

In some embodiments, the X-ray powder diffraction pattern of compound ATV-014 hydrochloride crystalline form II has characteristic peaks at 2Θ ± 0.2° values of 5.23, 9.88, 10.43, 14.05, 18.79 and 22.28, and further has two characteristic peaks at 2Θ ± 0.2° values of 15.68, 19.46 and 21.38.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 18.79, 19.46, 21.38 and 22.28.

In some embodiments, the X-ray powder diffraction pattern of compound ATV-014 hydrochloride crystalline form II has at least three characteristic peaks at 2Θ ± 0.2° values of 5.23, 9.88, 10.43, 14.05, 15.68, 18.79, 19.46, 21.38 and 22.28.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 18.79,19.46, 21.38 and 22.28, and further has one, two or three characteristic peaks at 2Θ ± 0.2° values of 17.81, 20.37 and 23.96.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 18.79, 19.46, 21.38 and 22.28, and further has one or two characteristic peaks at 2Θ ± 0.2° values of 17.81, 20.37 and 23.96.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 18.79, 19.46, 21.38 and 22.28, and further has two characteristic peaks at 2Θ ± 0.2° values of 17.81, 20.37 and 23.96.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 18.79, 19.46, 21.38 and 22.28, and further has one characteristic peak at 2Θ ± 0.2° values of 17.81, 20.37 and 23.96.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 17.81, 18.79, 19.46, 20.37, 21.38, 22.28 and 23.96.

In some embodiments, the X-ray powder diffraction pattern of compound ATV-014 hydrochloride crystalline form II has at least three characteristic peaks at 2Θ ± 0.2° values of 5.23, 9.88, 10.43, 14.05, 15.68, 17.81, 18.79, 19.46, 20.37, 21.38, 22.28 and 23.96.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 17.81, 18.79, 19.46, 20.37, 21.38, 22.28, and further has one or two characteristic peaks at 2Θ ± 0.2° values of 22.81 and 37.05.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 17.81, 18.79, 19.46, 20.37, 21.38, 22.28, and further has one characteristic peak at 2Θ ± 0.2° values of 22.81 and 37.05.

In some embodiments, the X-ray powder diffraction pattern of compound ATV-014 hydrochloride crystalline form II has characteristic peaks at 2Θ ± 0.2° values of 5.23, 9.88, 10.43, 14.05, 15.68, 17.81, 18.79, 19.46, 20.37, 21.38, 22.28, 22.81, 23.96 and 37.05.

In some embodiments, the X-ray powder diffraction pattern of compound ATV-014 hydrochloride crystalline form II has at least three characteristic peaks at 2Θ ± 0.2° values of 5.23, 9.88, 10.43, 14.05, 15.68, 17.81, 18.79, 19.46, 20.37, 21.38, 22.28, 22.81, 23.96 and 37.05.

### Compound ATV-014 hydrochloride crystalline form III

In some embodiments, there is provided compound ATV-014 hydrochloride crystalline form III, wherein the hydrochloride crystalline form III is a solvate.

In some embodiments, the hydrochloride crystalline form III has an X-ray powder diffraction pattern (XRPD pattern) substantially as the one shown in FIG. 4.

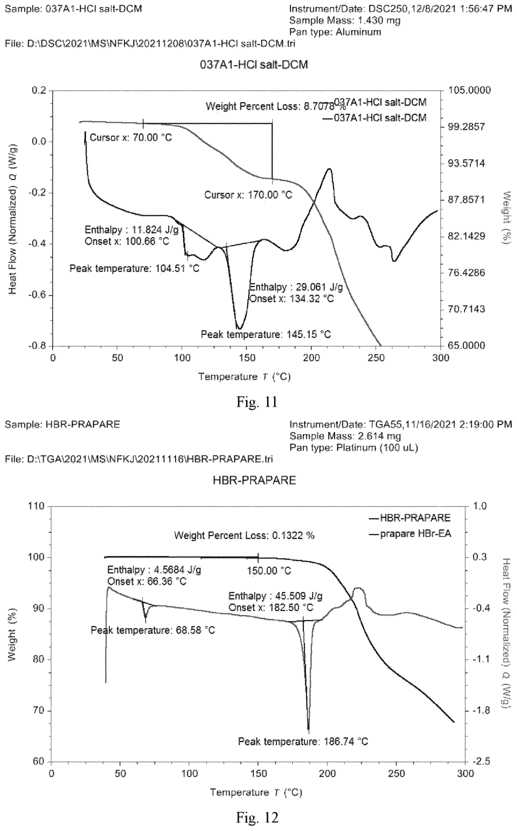

In some embodiments, the hydrochloride crystalline form III has a differential scanning calorimetry curve (DSC) substantially as the one shown in FIG. 11.

In some embodiments, the hydrochloride crystalline form III has a thermogravimetric analysis curve (TGA) substantially as the one shown in FIG. 11.

In some embodiments, compound ATV-014 hydrochloride crystalline form III is applicable to at least one, at least two, or all of the following (a) - (c): (a) crystalline form III has an X-ray powder diffraction pattern (XRPD pattern) substantially as depicted in FIG. 4; (b) crystalline form III has a differential scanning calorimetry curve (DSC) substantially as depicted in FIG. 11; (c) crystalline form III has a thermogravimetric analysis curve (TGA) substantially as depicted in FIG. 11.

In some embodiments, compound ATV-014 hydrochloride crystalline form III has the following properties:
(a) crystalline form III has an X-ray powder diffraction pattern (XRPD pattern) substantially as depicted in FIG. 4;
(b) crystalline form III has a differential scanning calorimetry curve (DSC) substantially as depicted in the differential scanning calorimetry curve in FIG. 11;
(c) crystalline form III has a thermogravimetric analysis curve (TGA) substantially as depicted in the thermogravimetric analysis curve in FIG. 11.

In some embodiments, compound ATV-014 hydrochloride crystalline form III has at least 2, at least 3, at least 4, at least 5, or at least 6 XRPD patterns with 2 θ degree reflections, and its maximum intensity is substantially as depicted in FIG.4.

In some embodiments, the differential scanning calorimetry curve of compound ATV-014 hydrochloride crystalline form III has an endothermic peak with a peak temperature of 90 °C - 170 °C. In some embodiments, the differential scanning calorimetry curve of the hydrochloride crystalline form III has an endothermic peak with a peak temperature 125 °C - 170 °C. In some embodiments, the differential scanning calorimetry curve of the hydrochloride crystalline form III has an endothermic peak with a peak temperature 90°C to 125°C, the endothermic peak is a desolvent peak. In some embodiments, the differential scanning calorimetry curve of the hydrochloride crystalline form III has an endothermic peak with a peak temperature of 101 °C to 120 °C and/or 130°C to 138°C. In some embodiments, the hydrochloride crystalline form III has a differential scanning calorimetry curve substantially as the one shown in FIG. 11.

In some embodiments, the differential scanning calorimetry curve of the hydrochloride crystalline form III shows its melting point as 134.32±5°C.

In some embodiments, the thermogravimetric analysis curve of the hydrochloride crystalline form III has a weight loss of less than 10% or less than 9% in a temperature range of 70°C tol70°C. In some embodiments, the thermogravimetric analysis curve of compound ATV014 hydrochloride crystalline form III has a weight loss of below 8.6% in a temperature range of 70 to 170°C. In some embodiments, the hydrochloride crystalline form III has a thermogravimetric analysis curve substantially as the one shown in FIG. 11.

In some embodiments, the X-ray powder diffraction pattern of compound ATV-014 hydrochloride crystalline form III has characteristic peaks at 2Θ ± 0.2° values of 5.28, 14.03 and 18.99.

In some embodiments, the X-ray powder diffraction pattern of compound ATV-014 hydrochloride crystalline form III has characteristic peaks at 2Θ ± 0.2° values of 5.28, 14.03 and 18.99, and further has one, two or three characteristic peaks at 2Θ ± 0.2° values of 10.54, 15.84 and 21.16.

In some embodiments, the X-ray powder diffraction pattern of compound ATV-014 hydrochloride crystalline form III has characteristic peaks at 2Θ ± 0.2° values of 5.28, 14.03 and 18.99, and further has one or two characteristic peaks at 2Θ ± 0.2° values of 10.54, 15.84 and 21.16.

In some embodiments, the X-ray powder diffraction pattern of compound ATV-014 hydrochloride crystalline form III has characteristic peaks at 2Θ ± 0.2° values of 5.28, 14.03 and 18.99, and further has one characteristic peak at 2Θ ± 0.2° values of 10.54, 15.84 and 21.16.

In some embodiments, the X-ray powder diffraction pattern of compound ATV-014 hydrochloride crystalline form III has characteristic peaks at 2Θ ± 0.2° values of 5.28, 14.03 and 18.99, and further has two characteristic peaks at 2Θ ± 0.2° values of 10.54, 15.84 and 21.16.

In some embodiments, the X-ray powder diffraction pattern of compound ATV-014 hydrochloride crystalline form III has characteristic peaks at 2Θ ± 0.2° values of 5.28, 10.54, 14.03, 15.84, 18.99 and 21.16.

In some embodiments, the X-ray powder diffraction pattern of compound ATV-014 hydrochloride crystalline form III has at least three characteristic peaks at 2Θ ± 0.2° values of 5.28, 10.54, 14.03, 15.84, 18.99 and 21.16.

In some embodiments, the X-ray powder diffraction pattern of compound ATV-014 hydrochloride crystalline form III has characteristic peaks at 2Θ ± 0.2° values of 5.28, 10.54, 14.03, 15.84, 18.99 and 21.16, and further has one, two or three characteristic peaks at 2Θ ± 0.2° values of 17.67, 22.49 and 27.51.

In some embodiments, the X-ray powder diffraction pattern of compound ATV-014 hydrochloride crystalline form III has characteristic peaks at 2Θ ± 0.2° values of 5.28, 10.54, 14.03, 15.84, 18.99 and 21.16, and further has one or two characteristic peaks at 2Θ ± 0.2° values of 17.67, 22.49 and 27.51.

In some embodiments, the X-ray powder diffraction pattern of compound ATV-014 hydrochloride crystalline form III has characteristic peaks at 2Θ ± 0.2° values of 5.28, 10.54, 14.03, 15.84, 18.99 and 21.16, and further has one characteristic peak at 2Θ ± 0.2° values of 17.67, 22.49 and 27.51.

In some embodiments, the X-ray powder diffraction pattern of compound ATV-014 hydrochloride crystalline form III has characteristic peaks at 2Θ ± 0.2° values of 5.28, 10.54, 14.03, 15.84, 18.99 and 21.16, and further has two characteristic peaks at 2Θ ± 0.2° values of 17.67, 22.49 and 27.51.

In some embodiments, the X-ray powder diffraction pattern of compound ATV-014 hydrochloride crystalline form III has characteristic peaks at 2Θ ± 0.2° values of 5.28, 10.54, 14.03, 15.84, 17.67, 18.99, 21.16, 22.49 and 27.51.

In some embodiments, the X-ray powder diffraction pattern of compound ATV-014 hydrochloride crystalline form III has at least three characteristic peaks at 2Θ ± 0.2° values of 5.28, 10.54, 14.03, 15.84, 17.67, 18.99, 21.16, 22.49 and 27.51.

### Compound ATV-014 hydrochloride crystalline form IV

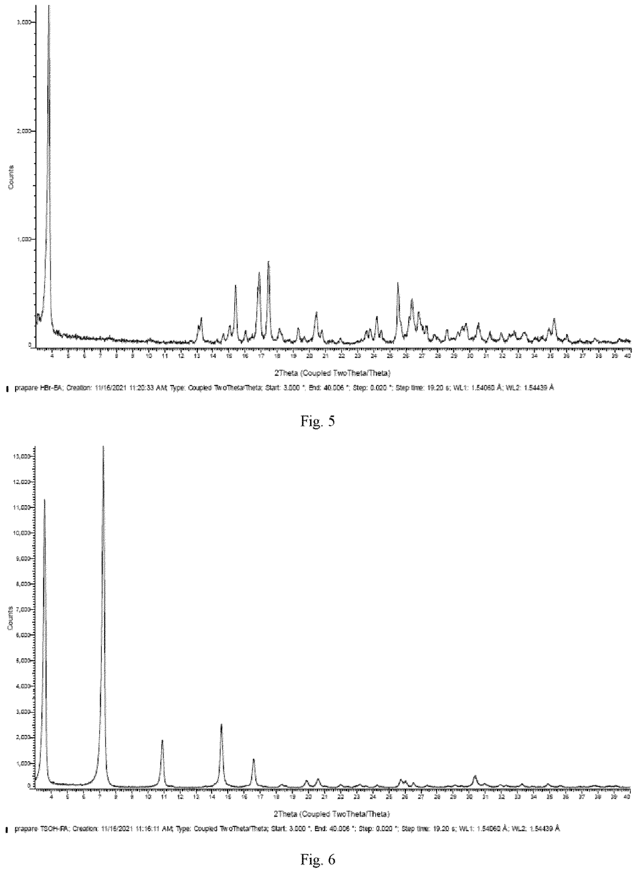

In some embodiments, the hydrochloride crystalline form IV has an X-ray powder diffraction pattern (XRPD pattern) substantially as the one shown in FIG. 5.

In some embodiments, the hydrochloride crystalline form IV has a differential scanning calorimetry curve (DSC) substantially as the one shown in FIG. 12.

In some embodiments, the hydrochloride crystalline form IV has a thermogravimetric analysis curve (TGA) substantially as the one shown in FIG. 12.

In some embodiments, compound ATV-014 hydrochloride crystalline form IV is applicable to at least one, at least two, or all of the following (a) - (c): (a) crystalline form IV has an X-ray powder diffraction pattern (XRPD pattern) substantially as depicted in FIG. 5; (b) crystalline form IV has a differential scanning calorimetry curve (DSC) substantially as depicted in FIG. 12; (c) crystalline form IV has a thermogravimetric analysis curve (TGA) substantially as depicted in FIG. 12.

In some embodiments, compound ATV-014 hydrochloride crystalline form IV has the following properties:
(a) crystalline form IV has an X-ray powder diffraction pattern (XRPD pattern) substantially as depicted in FIG. 5;
(b) crystalline form IV has a differential scanning calorimetry curve (DSC) substantially as depicted in the differential scanning calorimetry curve in FIG. 12;
(c) crystalline form IV has a thermogravimetric analysis curve (TGA) substantially as depicted in the thermogravimetric analysis curve in FIG. 12.

In some embodiments, the differential scanning calorimetry curve of the hydrochloride crystalline form IV has an endothermic peak with a peak temperature of 180 °C - 200 °C. In some embodiments, the differential scanning calorimetry curve of the hydrochloride crystalline form IV has an endothermic peak with a peak temperature of 183 °C - 190 °C. In some embodiments, the hydrochloride crystalline form IV has a differential scanning calorimetry curve substantially as the one shown in FIG. 12.

In some embodiments, the differential scanning calorimetry curve of the hydrochloride crystalline form IV shows its melting point as 182.50 ± 5°C.

In some embodiments, the thermogravimetric analysis curve of the hydrochloride crystalline form IV has a weight loss of less than 2% in a temperature range of 30 °C to 150 °C. In some embodiments, the thermogravimetric analysis curve of the hydrochloride crystalline form IV has a weight loss of less than 1% in a temperature range of 30 °C to 150 °C. In some embodiments, the thermogravimetric analysis curve of the hydrochloride crystalline form IV has a weight loss of less than 0.5% in a temperature range of 30 °C to 150 °C. In some embodiments, the thermogravimetric analysis curve of the hydrochloride crystalline form IV has a weight loss of less than 0.2% in a temperature range of 30 °C to 150 °C.

In some embodiments, compound ATV-014 hydrochloride crystalline form IV has at least 2, at least 3, at least 4, at least 5, or at least 6 XRPD patterns with 2Θ degree reflections, and its maximum intensity is substantially as depicted in FIG.5.

In some embodiments, the X-ray powder diffraction pattern of compound ATV-014 hydrochloride crystalline form IV has characteristic peaks at 2Θ ± 0.2° values of 5.28, 10.63 and 19.13.

In some embodiments, the X-ray powder diffraction pattern of compound ATV-014 hydrochloride crystalline form IV has characteristic peaks at 2Θ ± 0.2° values of 5.28, 10.63 and 19.13, and further has one, two or three characteristic peaks at 2Θ ± 0.2° values of 9.63, 14.10 and 16.82.

In some embodiments, the X-ray powder diffraction pattern of compound ATV-014 hydrochloride crystalline form IV has characteristic peaks at 2Θ ± 0.2° values of 5.28, 10.63 and 19.13, and further has one or two characteristic peaks at 2Θ ± 0.2° values of 9.63, 14.10 and 16.82.

In some embodiments, the X-ray powder diffraction pattern of compound ATV-014 hydrochloride crystalline form IV has characteristic peaks at 2Θ ± 0.2° values of 5.28, 10.63 and 19.13, and further has one characteristic peak at 2Θ ± 0.2° values of 9.63, 14.10 and 16.82.

In some embodiments, the X-ray powder diffraction pattern of compound ATV-014 hydrochloride crystalline form IV has characteristic peaks at 2Θ ± 0.2° values of 5.28, 10.63 and 19.13, and further has two characteristic peaks at 2Θ ± 0.2° values of 9.63, 14.10 and 16.82.

In some embodiments, the X-ray powder diffraction pattern of compound ATV-014 hydrochloride crystalline form IV has characteristic peaks at 2Θ ± 0.2° values of 5.28, 9.63, 10.63, 14.10, 16.82 and 19.13.

In some embodiments, the X-ray powder diffraction pattern of compound ATV-014 hydrochloride crystalline form IV has at least three characteristic peaks at 2Θ ± 0.2° values of 5.28, 9.63, 10.63, 14.10, 16.82 and 19.13.

In some embodiments, the X-ray powder diffraction pattern of compound ATV-014 hydrochloride crystalline form IV has characteristic peaks at 2Θ ± 0.2° values of 5.28, 9.63, 10.63, 14.10, 16.82 and 19.13, and further has one, two or three characteristic peaks at 2Θ ± 0.2° values of 10.19, 15.99 and 17.62.

In some embodiments, the X-ray powder diffraction pattern of compound ATV-014 hydrochloride crystalline form IV has characteristic peaks at 2Θ ± 0.2° values of 5.28, 9.63, 10.63, 14.10, 16.82 and 19.13, and further has one or two characteristic peaks at 2Θ ± 0.2° values of 10.19, 15.99 and 17.62.

In some embodiments, the X-ray powder diffraction pattern of compound ATV-014 hydrochloride crystalline form IV has characteristic peaks at 2Θ ± 0.2° values of 5.28, 9.63, 10.63, 14.10, 16.82 and 19.13, and further has one characteristic peak at 2Θ ± 0.2° values of 10.19, 15.99 and 17.62.

In some embodiments, the X-ray powder diffraction pattern of compound ATV-014 hydrochloride crystalline form IV has characteristic peaks at 2Θ ± 0.2° values of 5.28, 9.63, 10.63, 14.10, 16.82 and 19.13, and further has two characteristic peaks at 2Θ ± 0.2° values of 10.19, 15.99 and 17.62.

In some embodiments, the X-ray powder diffraction pattern of compound ATV-014 hydrochloride crystalline form IV has characteristic peaks at 2Θ ± 0.2° values of 5.28, 9.63, 10.19, 10.63, 14.10, 15.99, 16.82, 17.62 and 19.13.

In some embodiments, the X-ray powder diffraction pattern of compound ATV-014 hydrochloride crystalline form IV has at least three characteristic peaks at 2Θ ± 0.2° values of 5.28, 9.63, 10.19, 10.63, 14.10, 15.99, 16.82, 17.62 and 19.13.

In some embodiments, the X-ray powder diffraction pattern of compound ATV-014 hydrochloride crystalline form IV has characteristic peaks at 2Θ ± 0.2° values of 5.28, 9.63, 10.19, 10.63, 14.10, 15.99, 16.82, 17.62 and 19.13, and further has one, two or three characteristic peaks at 2Θ ± 0.2° values of 22.27, 23.19 and 27.74.

In some embodiments, the X-ray powder diffraction pattern of compound ATV-014 hydrochloride crystalline form IV has characteristic peaks at 2Θ ± 0.2° values of 5.28, 9.63, 10.19, 10.63, 14.10, 15.99, 16.82, 17.62 and 19.13, and further has one or two characteristic peaks at 2Θ ± 0.2° values of 22.27, 23.19 and 27.74.

In some embodiments, the X-ray powder diffraction pattern of compound ATV-014 hydrochloride crystalline form IV has characteristic peaks at 2Θ ± 0.2° values of 5.28, 9.63, 10.19, 10.63, 14.10, 15.99, 16.82, 17.62 and 19.13, and further has one characteristic peak at 2Θ ± 0.2° values of 22.27, 23.19 and 27.74.

In some embodiments, the X-ray powder diffraction pattern of compound ATV-014 hydrochloride crystalline form IV has characteristic peaks at 2Θ ± 0.2° values of 5.28, 9.63, 10.19, 10.63, 14.10, 15.99, 16.82, 17.62 and 19.13, and further has two characteristic peaks at 2Θ ± 0.2° values of 22.27, 23.19 and 27.74.

In some embodiments, the X-ray powder diffraction pattern of compound ATV-014 hydrochloride crystalline form IV has characteristic peaks at 2Θ ± 0.2° values of 5.28, 9.63, 10.19, 10.63, 14.10, 15.99, 16.82, 17.62, 19.13, 22.27, 23.19 and 27.74.

In some embodiments, the X-ray powder diffraction pattern of compound ATV-014 hydrochloride crystalline form IV has at least three characteristic peaks at 2Θ ± 0.2° values of 5.28, 9.63, 10.19, 10.63, 14.10, 15.99, 16.82, 17.62, 19.13, 22.27, 23.19 and 27.74.

In some embodiments, the X-ray powder diffraction pattern of compound ATV-014 hydrochloride crystalline form IV has characteristic peaks at 2Θ ± 0.2° values of 5.28, 9.63, 10.19, 10.63, 14.10, 15.99, 16.82, 17.62, 19.13, 22.27, 22.56, 23.19 and 27.74.

In some embodiments, the X-ray powder diffraction pattern of compound ATV-014 hydrochloride crystalline form IV has at least three characteristic peaks at 2Θ ± 0.2° values of 5.28, 9.63, 10.19, 10.63, 14.10, 15.99, 16.82, 17.62, 19.13, 22.27, 22.56, 23.19 and 27.74.

### Compound ATV-014 hydrobromide crystalline form I

In some embodiments, there is provided compound ATV-014 hydrobromide crystalline form I, wherein the hydrobromide crystalline form I is an anhydrous crystal.

In some embodiments, the hydrobromide crystalline form I has an X-ray powder diffraction pattern (XRPD pattern) substantially as the one shown in FIG. 7.

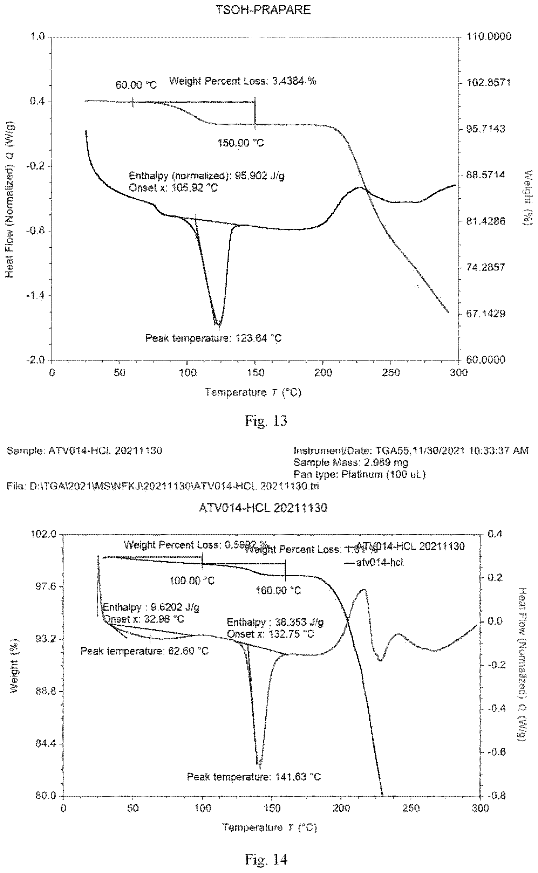

In some embodiments, the hydrobromide crystalline form I has a differential scanning calorimetry curve (DSC) substantially as the one shown in FIG. 14.

In some embodiments, the hydrobromide crystalline form I has a thermogravimetric analysis curve (TGA) substantially as the one shown in FIG. 14.

In some embodiments, compound ATV-014 hydrobromide crystalline form I is applicable to at least one, at least two, or all of the following (a) - (c): (a) crystalline form I has an X-ray powder diffraction pattern (XRPD pattern) substantially as depicted in FIG. 7; (b) crystalline form I has a differential scanning calorimetry curve (DSC) substantially as depicted in FIG. 14; (c) crystalline form I has a thermogravimetric analysis curve (TGA) substantially as depicted in FIG. 14.

In some embodiments, compound ATV-014 hydrobromide crystalline form I has the following properties:
(a) crystalline form I has an X-ray powder diffraction pattern (XRPD pattern) substantially as depicted in FIG. 7;
(b) crystalline form I has a differential scanning calorimetry curve (DSC) substantially as depicted in the differential scanning calorimetry curve in FIG. 14;
(c) crystalline form I has a thermogravimetric analysis curve (TGA) substantially as depicted in the thermogravimetric analysis curve in FIG. 14.

In some embodiments, the compound ATV-014 hydrobromide crystalline form I has at least 2, at least 3, at least 4, at least 5, or at least 6 XRPD patterns with 2 θ degree reflections, and its maximum intensity is substantially as depicted in FIG.7.

In some embodiments, the thermogravimetric analysis curve of compound ATV014 hydrobromide crystalline form I has a weight loss of less than 2% in a temperature range of 100 °C to 160 °C. In some embodiments, the thermogravimetric analysis curve of compound ATV014 hydrobromide crystalline form I has a weight loss of less than 1.5% in a temperature range of 100 °C to 160 °C. In some embodiments, the compound ATV-014 hydrobromide crystalline form I has a thermogravimetric analysis curve substantially as the one shown in FIG. 14.

In some embodiments, the thermogravimetric analysis curve of compound ATV014 hydrobromide crystalline form I has no significant weight loss in a temperature of -170°C. In some embodiments, the differential scanning calorimetry curve of compound ATV-014 hydrobromide crystalline form I has an endothermic peak with a peak temperature of 130 °C - 170 °C. In some embodiments, the differential scanning calorimetry curve of compound ATV-014 hydrobromide crystalline form I has an endothermic peak with a peak temperature of 138 °C - 145 °C. In some embodiments, the differential scanning calorimetry curve of compound ATV-014 hydrobromide crystalline form I has an endothermic peak with a peak temperature of 59 °C- 66 °C, the endothermic peak is a desolvent peak. In some embodiments, compound ATV-014 hydrobromide crystalline form I has a differential scanning calorimetry curve substantially as the one shown in FIG. 14.

In some embodiments, the thermogravimetric analysis curve of compound ATV-014 hydrobromide crystalline form I shows that the melting point is 132.75 ± 5 °C.

In some embodiments, the differential scanning calorimetry curve of compound ATV-014 hydrobromide crystalline form I has endothermic peaks with peak temperatures of 64 °C - 66 °C and 179 °C - 183 °C.

In some embodiments, the X-ray powder diffraction pattern of compound ATV-014 hydrobromide crystalline form I has characteristic peaks at 2Θ ± 0.2° values of 3.78, 13.20 and 16.88.

In some embodiments, the X-ray powder diffraction pattern of compound ATV-014 hydrobromide crystalline form I has characteristic peaks at 2Θ ± 0.2° values of 3.78, 13.20 and 16.88, and further has one, two or three characteristic peaks at 2Θ ± 0.2° values of 15.41, 17.46 and 26.38.

In some embodiments, the X-ray powder diffraction pattern of compound ATV-014 hydrobromide crystalline form I has characteristic peaks at 2Θ ± 0.2° values of 3.78, 13.20 and 16.88, and further has one or two characteristic peaks at 2Θ ± 0.2° values of 15.41, 17.46 and 26.38.

In some embodiments, the X-ray powder diffraction pattern of compound ATV-014 hydrobromide crystalline form I has characteristic peaks at 2Θ ± 0.2° values of 3.78, 13.20 and 16.88, and further has one characteristic peak at 2Θ ± 0.2° values of 15.41, 17.46 and 26.38.

In some embodiments, the X-ray powder diffraction pattern of compound ATV-014 hydrobromide crystalline form I has characteristic peaks at 2Θ ± 0.2° values of 3.78, 13.20 and 16.88, and further has two characteristic peaks at 2Θ ± 0.2° values of 15.41, 17.46 and 26.38.

In some embodiments, the X-ray powder diffraction pattern of compound ATV-014 hydrobromide crystalline form I has characteristic peaks at 2Θ ± 0.2° values of 3.78, 13.20, 15.41, 16.88, 17.46 and 26.38.

In some embodiments, the X-ray powder diffraction pattern of compound ATV-014 hydrobromide crystalline form I has at least three characteristic peaks at 2Θ ± 0.2° values of 3.78, 13.20, 15.41, 16.88, 17.46 and 26.38.

In some embodiments, the X-ray powder diffraction pattern of compound ATV-014 hydrobromide crystalline form I has characteristic peaks at 2Θ ± 0.2° values of 3.78, 13.20, 15.41, 16.88, 17.46 and 26.38, and further has one or two characteristic peaks at 2Θ ± 0.2° values of 25.52 and 26.79.

In some embodiments, the X-ray powder diffraction pattern of compound ATV-014 hydrobromide crystalline form I has characteristic peaks at 2Θ ± 0.2° values of 3.78, 13.20, 15.41, 16.88, 17.46 and 26.38, and further has one characteristic peak at 2Θ ± 0.2° values of 25.52 and 26.79.

In some embodiments, the X-ray powder diffraction pattern of compound ATV-014 hydrobromide crystalline form I has characteristic peaks at 2Θ ± 0.2° values of 3.78, 13.20, 15.41, 16.88, 17.46, 25.52, 26.38 and 26.79.

In some embodiments, the X-ray powder diffraction pattern of compound ATV-014 hydrobromide crystalline form I has at least three characteristic peaks at 2Θ ± 0.2° values of 3.78, 13.20, 15.41, 16.88, 17.46, 25.52, 26.38 and 26.79.

### Compound ATV-014 p-toluenesulfonate crystalline form I

In some embodiments, there is provided compound ATV-014 *p*-toluenesulfonate crystalline form I, wherein the *p*-toluenesulfonate crystalline form I is a monohydrate.

In some embodiments, the *p*-toluenesulfonate crystalline form I has an X-ray powder diffraction pattern (XRPD pattern) substantially as the one shown in FIG. 6.

In some embodiments, the *p*-toluenesulfonate crystalline form I has a differential scanning calorimetry curve (DSC) substantially as the one shown in FIG. 13.

In some embodiments, the *p*-toluenesulfonate crystalline form I has a thermogravimetric analysis curve (TGA) substantially as the one shown in FIG. 13.

In some embodiments, compound ATV-014 *p*-toluenesulfonate crystalline form I is applicable to at least one, at least two, or all of the following (a) - (c): (a) crystalline form I has an X-ray powder diffraction pattern (XRPD pattern) substantially as depicted in FIG. 6; (b) crystalline form I has a differential scanning calorimetry curve (DSC) substantially as depicted in FIG. 13; (c) crystalline form I has a thermogravimetric analysis curve (TGA) substantially as depicted in FIG. 13.

In some embodiments, compound ATV-014 *p*-toluenesulfonate crystalline form I has the following properties:
(a) crystalline form I has an X-ray powder diffraction pattern (XRPD pattern) substantially as depicted in FIG. 6;
(b) crystalline form I has a differential scanning calorimetry curve (DSC) substantially as depicted in the differential scanning calorimetry curve in FIG. 13;
(c) crystalline form I has a thermogravimetric analysis curve (TGA) substantially as depicted in the thermogravimetric analysis curve in FIG. 13.

In some embodiments, the compound ATV-014 *p*-toluenesulfonate crystalline form I has at least 2, at least 3, at least 4, at least 5, or at least 6 XRPD patterns with 2 θ degree reflections, and its maximum intensity is substantially as depicted in FIG.6.

In some embodiments, the thermogravimetric analysis curve of compound ATV014 *p*-toluenesulfonate crystalline form I has a weight loss of less than 10% in a temperature range of 60 °C to 150 °C. In some embodiments, the thermogravimetric analysis curve of compound ATV014 *p*-toluenesulfonate crystalline form I has a weight loss of less than 5% in a temperature range of 60 °C to 150 °C. In some embodiments, the thermogravimetric analysis curve of compound E1TV014 *p*-toluenesulfonate crystalline form I has a weight loss of less than 4% in a temperature range of 60°C to 150 °C. In some embodiments, the compound ATV-014 *p*-toluenesulfonate crystalline form I has a thermogravimetric analysis curve substantially as the one shown in FIG. 13.

In some embodiments, the thermogravimetric analysis curve of compound ATV-014 *p*-toluenesulfonate crystalline form I shows that the melting point is 105.92 ± 5 °C.

In some embodiments, the differential scanning calorimetry curve of compound ATV-014 *p*-toluenesulfonate crystalline form I has an endothermic peak with a peak temperature of 100 °C - 140 °C. In some embodiments, the differential scanning calorimetry curve of compound ATV-014 *p*-toluenesulfonate crystalline form I has an endothermic peak with a peak temperature of 120 °C - 127 °C. In some embodiments, the differential scanning calorimetry curve of compound ATV-014 *p*-toluenesulfonate crystalline form I has an endothermic peak with a peak temperature of 121 °C- 126 °C, the enthalpy value is 66 J/g. In some embodiments, compound ATV-014 *p*-toluenesulfonate crystalline form I has a differential scanning calorimetry curve substantially as the one shown in FIG. 13.

In some embodiments, the thermogravimetric analysis curve of compound ATV014 *p*-toluenesulfonate crystalline form I has a weight loss of below 3.2% in a temperature range of 65 °C to 130 °C.

In some embodiments, the X-ray powder diffraction pattern of compound ATV-014 *p*-toluenesulfonate crystalline form I has characteristic peaks at 2Θ ± 0.2° values of 3.58, 10.89 and 16.59.

In some embodiments, the X-ray powder diffraction pattern of compound ATV-014 *p*-toluenesulfonate crystalline form I has characteristic peaks at 2Θ ± 0.2° values of 3.58, 10.89 and 16.59, and further has one or two characteristic peaks at 2Θ ± 0.2° values of 7.23 and 14.59.

In some embodiments, the X-ray powder diffraction pattern of compound ATV-014 *p*-toluenesulfonate crystalline form I has characteristic peaks at 2Θ ± 0.2° values of 3.58, 10.89 and 16.59, and further has one characteristic peak at 2Θ ± 0.2° values of 7.23 and 14.59.

In some embodiments, the X-ray powder diffraction pattern of compound ATV-014 *p*-toluenesulfonate crystalline form I has characteristic peaks at 2Θ ± 0.2° values of 3.58, 7.23, 10.89, 14.59 and 16.59.

In some embodiments, the X-ray powder diffraction pattern of compound ATV-014 *p*-toluenesulfonate crystalline form I has at least three characteristic peaks at 2Θ ± 0.2° values of 3.58, 7.23, 10.89, 14.59 and 16.59.
The novel crystalline forms of compound ATV014 free base and its salts described herein can be used for treating viral infection related diseases, or for preventing, lessening, and/or treating symptoms of SARS-CoV-2 related diseases.

In second aspect, the present invention provides a pharmaceutical composition.

Another objective of the present invention is to provide a pharmaceutical composition containing a therapeutically effective amount of a novel crystalline form of compound ATV014 free base and its salts, as well as a pharmaceutically acceptable an adjuvant or excipient. Generally, a therapeutically effective amount of the novel crystalline form of compound ATV014 free base and its salts is mixed or contacted with one or more medicinal adjuvants to form a pharmaceutical composition or formulation. The pharmaceutical composition or formulation is prepared in a manner well-known in the pharmaceutical field. The pharmaceutical composition or formulation can be used for treating viral infection related diseases, or for preventing, lessening, and/or treating symptoms of SARS-CoV-2 related diseases.

In some embodiments, a pharmaceutical composition comprises a therapeutically effective amount of compound ATV014 hydrochloride crystalline form I, a therapeutically effective amount of compound ATV014 hydrochloride crystalline form II, a therapeutically effective amount of compound ATV014 hydrochloride crystalline form III, a therapeutically effective amount of compound ATV014 hydrochloride crystalline form IV, a therapeutically effective amount of compound ATV014 hydrobromate crystalline form I or a therapeutically effective amount of compound ATV014 *p*-toluenesulfonate crystalline form I, and optionally a pharmaceutically acceptable excipient.

In some embodiments, a pharmaceutical composition comprises a compound ATV014 free base, wherein at least 80% of the compound ATV014 free base is the compound ATV014 free base crystalline form I. In some embodiments, a pharmaceutical composition comprises a compound ATV014 free base, wherein at least 90% of the compound ATV014 free base is the compound ATV014 free base crystalline form I. In some embodiments, a pharmaceutical composition comprises a compound ATV014 free base, wherein at least 95% of the compound ATV014 free base is the compound ATV014 free base crystalline form I. In some embodiments, a pharmaceutical composition comprises a compound ATV014 free base, wherein at least 99% of the compound ATV014 free base is the compound ATV014 free base crystalline form I.

In some embodiments, a pharmaceutical composition comprises a compound ATV014 hydrochloride, wherein at least 80% of the compound ATV014 hydrochloride is the compound ATV014 hydrochloride crystalline form I. In some embodiments, a pharmaceutical composition comprises a compound ATV014 hydrochloride, wherein at least 90% of the compound ATV014 hydrochloride is the compound ATV014 hydrochloride crystalline form I. In some embodiments, a pharmaceutical composition comprises a compound ATV014 hydrochloride, wherein at least 95% of the compound ATV014 hydrochloride is the compound ATV014 hydrochloride crystalline form I. In some embodiments, a pharmaceutical composition comprises a compound ATV014 hydrochloride, wherein at least 99% of the compound ATV014 hydrochloride is the compound ATV014 hydrochloride crystalline form I.

In some embodiments, a pharmaceutical composition comprises a compound ATV014 hydrochloride, wherein at least 80% of the compound ATV014 hydrochloride is the compound ATV014 hydrochloride crystalline form II. In some embodiments, a pharmaceutical composition comprises a compound ATV014 hydrochloride, wherein at least 90% of the compound ATV014 hydrochloride is the compound ATV014 hydrochloride crystalline form II. In some embodiments, a pharmaceutical composition comprises a compound ATV014 hydrochloride, wherein at least 95% of the compound ATV014 hydrochloride is the compound ATV014 hydrochloride crystalline form II. In some embodiments, a pharmaceutical composition comprises a compound ATV014 hydrochloride, wherein at least 99% of the compound ATV014 hydrochloride is the compound ATV014 hydrochloride crystalline form II.

In some embodiments, a pharmaceutical composition comprises a compound ATV014 hydrochloride, wherein at least 80% of the compound ATV014 hydrochloride is the compound ATV014 hydrochloride crystalline form III. In some embodiments, a pharmaceutical composition comprises a compound ATV014 hydrochloride, wherein at least 90% of the compound ATV014 hydrochloride is the compound ATV014 hydrochloride crystalline form III. In some embodiments, a pharmaceutical composition comprises a compound ATV014 hydrochloride, wherein at least 95% of the compound ATV014 hydrochloride is the compound ATV014 hydrochloride crystalline form III. In some embodiments, a pharmaceutical composition comprises a compound ATV014 hydrochloride, wherein at least 99% of the compound ATV014 hydrochloride is the compound E1TV014 hydrochloride crystalline form III.

In some embodiments, a pharmaceutical composition comprises a compound ATV014 hydrochloride, wherein at least 80% of the compound ATV014 hydrochloride is the compound ATV014 hydrochloride crystalline form IV In some embodiments, a pharmaceutical composition comprises a compound ATV014 hydrochloride, wherein at least 90% of the compound ATV014 hydrochloride is the compound ATV014 hydrochloride crystalline form IV In some embodiments, a pharmaceutical composition comprises a compound ATV014 hydrochloride, wherein at least 95% of the compound ATV014 hydrochloride is the compound ATV014 hydrochloride crystalline form IV In some embodiments, a pharmaceutical composition comprises a compound ATV014 hydrochloride, wherein at least 99% of the compound ATV014 hydrochloride is the compound E1TV014 hydrochloride crystalline form IV

In some embodiments, a pharmaceutical composition comprises a compound ATV014 hydrobromide, wherein at least 80% of the compound ATV014 hydrobromide is the compound ATV014 hydrobromide crystalline form I. In some embodiments, a pharmaceutical composition comprises a compound ATV014 hydrobromide, wherein at least 90% of the compound ATV014 hydrobromide is the compound ATV014 hydrobromide crystalline form I. In some embodiments, a pharmaceutical composition comprises a compound ATV014 hydrobromide, wherein at least 95% of the compound ATV014 hydrobromide is the compound ATV014 hydrobromide crystalline form I. In some embodiments, a pharmaceutical composition comprises a compound ATV014 hydrobromide, wherein at least 99% of the compound ATV014 hydrobromide is the compound E1TV014 hydrobromide crystalline form I.

In some embodiments, a pharmaceutical composition comprises a compound E1TV014 *p*-toluenesulfonate, wherein at least 80% of the compound ATV014 *p*-toluenesulfonate is the compound ATV014 *p*-toluenesulfonate crystalline form I. In some embodiments, a pharmaceutical composition comprises a compound E1TV014 *p*-toluenesulfonate, wherein at least 90% of the compound E1TV014 *p*-toluenesulfonate is the compound ATV014 *p*-toluenesulfonate crystalline form I. In some embodiments, a pharmaceutical composition comprises a compound ATV014 *p*-toluenesulfonate, wherein at least 95% of the compound ATV014 *p*-toluenesulfonate is the compound ATV014 *p*-toluenesulfonate crystalline form I. In some embodiments, a pharmaceutical composition comprises a compound ATV014 *p*-toluenesulfonate, wherein at least 99% of the compound ATV014 *p*-toluenesulfonate is the compound ATV014 *p*-toluenesulfonate crystalline form I.

In third aspect, the present invention provides use of the aforementioned crystalline form or the aforementioned pharmaceutical composition.

Use of compound ATV014 hydrochloride crystalline form I, compound ATV014 hydrochloride crystalline form II, compound ATV014 hydrochloride crystalline form III, compound ATV014 hydrochloride crystalline form IV, compound ATV014 hydrobromate crystalline form I or compound ATV014 *p*-toluenesulfonate crystalline form I of the first aspect, or pharmaceutical composition of the second aspect in the manufacture of a medicament for treating a disease or symptom related to viral infections.

The novel crystalline forms containing compound ATV014 free base and its salts described herein can be used for preventing, lessening, and/or treating viral infection related diseases, or for preventing, lessening, and/or treating symptoms of SARS-CoV-2 related diseases.

The novel crystalline forms provided herein have not been reported yet. After research, the inventors of the present invention have overcome this challenge and found a suitable novel crystalline forms for development. These novel crystalline forms have better stability and solubility, and a more stable crystalline form is of great significance for improving drug quality.

The novel crystalline forms provided herein have good stability in water, is not easily deliquescent under high humidity conditions, and is convenient for long-term storage and placement of drugs. The crystalline forms provided herein have good stability, significant effect of process purification, and can effectively avoid crystal transformation during drug storage or the development process, thereby avoiding changes in bioavailability and drug efficacy, and has strong economic value.

### Beneficial Effects of the invention:

(1) The compound ATV-014 hydrobromate crystalinel form I provided herein is placed under 70% RH (open) conditions for one week, the moisture absorption is 0.2%, and under 90% RH (open) conditions for one week, the moisture absorption is 18.8%.
(2) The compound ATV-014 *p*-toluenesulfonate crystalline form I provided herein is placed under 30% RH (open) conditions for one week, the moisture absorption is 0.4%, and under 90% RH (open) conditions for one week, the moisture absorption is 0.5%. The crystalline structure and chemical purity have not changed, and the physical and chemical stability is good.
(3) The DVS results indicate that compound ATV-014 hydrobromate crystalline form I has hygroscopicity; After equilibrating for 30 to 120 minutes under 70% RH conditions, its hygroscopic weight gain was 0.2%; Under conditions ranging from 70% RH to 90% RH, the moisture absorption is strong, and the weight gain under 90% RH is 18.8%. The sample transforms into an amorphous glassy solid during the DVS testing process.
(4) The DVS results indicate that compound ATV-014 *p*-toluenesulfonate crystalline form I has slight hygroscopicity; After equilibrating for 30 to 120 minutes under 90% RH conditions, its hygroscopic weight gain was 0.5%. After the DVS test, there was no change in the crystalline structure of the remaining solid.
(5) The compound ATV-014 hydrochloride crystalline form provided herein is placed under 40 °C/75% RH (open) conditions for one week, the crystalline structure and chemical purity have not changed, and the physical and chemical stability is good.

### Definitions

Unless otherwise indicated, the following terms and phrases as used herein are intended to have the following meanings
The term "crystalline form" refers to a unique ordered arrangement and/or conformation of molecules in the lattice of a compound.

X-ray powder diffraction (XRPD) can detect changes in crystalline form, crystallinity, crystalline state and other information. It is a common mean for identifying crystalline form. The peak position of the XRPD pattern primarily depends on the structure of the crystalline form and is relatively insensitive to the experimental details, and its relative peak height depends on many factors associated with sample preparation and instrument geometry. Thus, in some embodiments, the crystalline form of the present invention is characterized by an XRPD pattern having certain peak positions, which is substantially as shown in the XRPD pattern provided in the drawings of the present invention. At the same time, the measurement of 2Θ of the XRPD pattern can have experimental errors, the measurement of 2Θ of the XRPD pattern may be slightly different between the different instruments and the different samples. Therefore, the values of 2Θ cannot be regarded as absolute. According to the condition of the instrument used in this test, the diffraction peak has an error tolerance of ±0.2°.

Differential Scanning Calorimetry (DSC) is a technique of measuring the energy difference between a sample and an inert reference (commonly used α-Al₂O₃) with temperature by continuously heating or cooling under program control. The endothermic peak height of the DSC curve depends on many factors associated with sample preparation and instrument geometry, while the peak position is relatively insensitive to experimental details. Thus, in some embodiments, the crystal form of the present invention is characterized by an DCS pattern having certain peak positions, which is substantially as shown in the DCS pattern provided in the drawings of the present invention. At the same time, the DCS pattern can have experimental errors, the peak positions and peak values of DCS pattern may be slightly different between the different instruments and the different samples. Therefore, the peak positions or the peak values of the DSC endothermic peak cannot be regarded as absolute. According to the condition of the instrument used in this test, the endothermic peak has an error tolerance of ±3°.

Glass state transition refers to the transition of amorphous substances between a high elastic state and a glass state, which is an inherent property of the substance; Its corresponding transition temperature is the glass transition temperature (Tg), which is an important physical property of amorphous materials. Glass transition is a phenomenon related to molecular motion, therefore, the glass transition temperature (Tg) mainly depends on the structure of the substance and is relatively insensitive to experimental details. According to the condition of the instrument used in this test, the endothermic peak has an error tolerance of ±3°.

Thermogravimetric analysis (TGA) is a technique for measuring the quality of a substance with temperature under the control of a program. It is suitable for examining the process of the solvent loss or the samples sublimation and decomposition. It can be presumed that the crystalline contains crystalline water or crystallization solvent. The quality variety of the TGA curve shown depend on a number of factors, including the sample preparation and the instrument. The quality variety of the TGA test may be slightly different between the different instruments and between the different samples. According to the condition of the instrument used in this test, there is a ±0.1% error tolerance for the mass change.

In the context of the present invention, the values of 2Θ in the X-ray powder diffraction pattern are all in degrees (°).

The "peak" refers to a feature that a person skilled in the art can recognize without belonging to background noise when referring to a spectrum or/and data that appears in the figure.

The term "essentially pure" refers that a crystalline form is substantially free of one or more other crystalline forms, the purity is at least 60%, or at least 70%, or at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or crystalline form containing other crystalline form. The percentage of the other crystalline form in the total volume or total weight of the crystalline form is less than 20%, or less than 10%, or less than 5%, or less than 3%, or less than 1%, or less than 0.5%, or less than 0.1%, or less than 0.01%.

The term "substantially free of one or more other crystalline forms" refers that the percentage of other crystalline forms in the total volume or total weight is less than 20%, or less than 10%, or less than 5%, or less than 4%, or less than 3%, or less than 2%, or less than 1%, or less than 0.5%, or less than 0.1%, or less than 0.01%.

The term "X-ray powder diffraction pattern is substantially as depicted in Fig." means at least 50%, or at least 60%, or at least 70%, or at least 80%, or at least 90%, or at least 95%, or at least 99% of the characteristic peaks shown in the X-ray powder diffraction pattern appear in the given pattern.

The term "relative intensity" refers to the ratio of the intensity of the other peaks to the intensity of the first strong peak in the X-ray powder diffraction pattern when the intensity of the first strong peak is 100%.

The term "anti-solvent" refers to a solvent that can promote a solution to achieve supersaturation or crystallization. In some embodiments, the solubility of ATV014 free base in the antisolvent is less than 0.001 g/L, or less than 0.01 g/L, or less than 0.1 g/L, or less than 0.2 g/L, or less than 0.3 g/L, or less than 0.4 g/L, or less than 0.5 g/L, or less than 0.6 g/L, or less than 0.8 g/L, or less than 1 g/L, or less than 2 g/L, or less than 3 g/L, or less than 4 g/L, or less than 5 g/L, or less than 6 g/L, or less than 7 g/L, or less than 8 g/L, or less than 9 g/L, or less than 10 g/L.

The "peak" refers to a featurethat is not attributed to background noise by those skilled in the art when referring to a spectrum or/and data in the figure.

The terms "option", "optional", or "optionally" refer to events or situations that may or may not necessarily occur subsequently described. For example, "optional pharmaceutically acceptable excipients" indicates that "pharmaceutically acceptable excipients" may or may not exist.

The numbers in this invention are approximate values, whether or not the words "approximately" or "approximately" are used. There may be differences of 1%, 2%, 5%, 7%, 8%, 10%, 15%, or 20% and so on in a number. Whenever a number having a value N is disclosed, any number having the value N+/-1%, N+/-2%, N+/-3%, N+/-5%, N+/-7%, N+/-8%, N+/-10%, N+/-15%, or N+/-20% values will be explicitly disclosed, where "+/-" refers to plus or minus. Whenever a numerical range with a lower limit, R^{L}, and an upper limit, R^{U}, is disclosed, any number falling within the range is specifically disclosed. Especially, it includes the following clearly disclosed values within this range: R=R^{L}+K*(R^{U}-R^{L}), wherein K is a variable that increases from 1% to 100% in increments of 1%, ie. 1%, 2%, 3%, 4%, 5%, 50%, 51%, 52%······95%, 96%, 97%, 98%, 99% or 100%. Moreover, a numerical range defined by two R numbers as defined above is also specifically disclosed. In the present invention, "room temperature" refers to the ambient temperature, which ranges from approximately 10 °C to approximately 40 °C. In some embodiments, "room temperature" refers to a temperature ranging from approximately 20 °C to approximately 30 °C; In other embodiments, "room temperature" refers to a temperature ranging from approximately 25 °C to approximately 30 °C; In some other embodiments, "room temperature" refers to 10 °C, 15 °C, 20 °C, 25 °C, 30 °C, 35 °C, 40 °C, etc.

The term "treating", as used herein, unless otherwise indicated, means reversing, lessening the disorder or condition to which such term applies, or one or more symptoms of such disorder or condition, or inhibiting the progression of the disorder or condition or one or more symptoms of such disorder or condition, or preventing the disorder or condition or one or more symptoms of such disorder or condition. The term "treatment", as used herein, refers to the act of treating, as "treating" is defined immediately above.

In the context of the present invention, "ATV-014 hydrobromate crystalline form I" and "compound ATV-014 hydrobromate crystalline form I" have the same meaning, and so on for other crystalline forms.

### Instrument parameters

Unless otherwise specified in the parameters description, all analyses below are conducted at room temperature.

### X-ray powder diffraction studies

X-ray powder diffraction patterns were collected by using a zero-background sample disk on a German Bruker D8 Advance X-ray diffractometer equipped with an automatic sampler. The used radiation source is Cu k α (λ=1.5418 Å), and the light tube voltage is set at 40KV and the light tube current is set at 40mA. The divergent slit of X-rays is 0.6mm. A proper amount of sample was placed in the center of the zero-background sample tray under the environmental condition, the sample was lightly pressed with a clean glass slide to obtain a flat plane, and the zero-background sample rack was fixed. Diffraction analysis of the sample was performed with a scanning step size of 0.02 °(2θ) in the range of 3-40° (2θ). The software used for data collection is DIFFRAC.COMMANDER, and the data is analyzed and presented using DIFFRAC.EVA.

### Differential scanning calorimetry (DSC)

DSC analysis of the sample was performed on a TA Instrument Discovery DSC 250 instrument and using a covered aluminum plate with holes. The sample (approximately 2-5 mg) was weighed on an aluminum plate, covering with Tzero, the sample amount was accurately recorded, and the sample was transferred to the instrument for measurement. The instrument is purged with nitrogen at a rate of 50 mL/min. Data was collected at a heating rate of 10 °C/min between 25 °C and 300 °C. The data was analyzed and presented using TRIOS.

### Thermogravimetric analysis (TGA)

TGA analysis of the sample was performed on a TA Instrument Discovery TGA 55 instrument and using an open aluminum plate. Typically, 2-5 mg of the sample was placed in a pre-equilibrated aluminum sample tray and heated from ambient temperature to 300 °C at a rate of 10 °C/min. A nitrogen flow of 25 mL/min maintains in the sample room. In the TGA spectrum, the abscissa represents temperature (°C), and the ordinate represents the weight loss percentage (Weight (%)).

### High performance liquid chromatography (HPLC) detection of crystalline forms

The instrument used for liquid chromatography analysis is Agilent HPLC 1260 series,

**Table1. HPLC method for stability research**

| | | | | |
|---|---|---|---|---|
| **Chromatographic column** | Agilent XDB-C18, 1.8 µm, 4.6*50 mm | | | |
| **Mobile phase** | A: 0.1% aqueous solution of trifluoroacetic acid; B: 0.1% trifluoroacetic acid acetonitrile solution | | | |
| **Gradient (T/BO/O)** | time | 0 min | 8 min | 10 min |
| | mobile phase A | 90% | 25% | 0% |
| | mobile phase B | 10% | 75% | 100% |
| **Column temperature** | 40 °C | | | |
| **Detector** | DAD detector, wavelength: 237 nm | | | |
| **Flow rate** | 1.0 mL/min | | | |
| **Injection volume** | 2.0 µL | | | |
| **Run time** | 10 min | | | |
| **Previous running time** | 3 min | | | |
| **Diluent** | acetonitrile: water=1:1 | | | |

The above-mentioned drugs in various formulations can be prepared according to conventional methods in the pharmaceutical field.

The abbreviations of some compounds in the present invention, Table 2:

| **Abbreviations** | **Chemical structure** |
|---|---|
| compound ATV-014 | 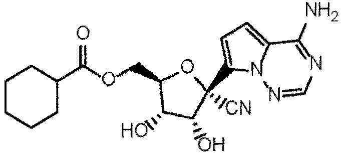 |

When describing the details of the experiment, certain abbreviations and abbreviated words were used. Although most of them can be understood by technical personnel in this field, the following table contains a list of these abbreviations and abbreviated words, Table 3

| **Abbreviations** | **Meaning** |
|---|---|
| ACN | acetonitrile |
| DCC | dicyclohexylcarbodiimide |
| DCM | dichloromethane |
| DMAP | 4-dimethylaminopyridine |
| EDMA | N,N-dimethylethylamine |
| PE | petroleum ether |
| rt | room temperature |
| TEA | triethylamine |
| TLC | thin layer chromatography |
| MeOH | methanol |
| EtOH | ethanol |
| IPA | isopropanol |
| NBA | n-butanol |
| MEK | butanone |
| MTBE | methyl tert-butyl ether |
| EA | ethyl acetate |
| IPAc | isopropyl acetate |
| Tol | toluene |
| Hept | n-heptane |
| THF | tetrahydrofuran |
| DMSO | dimethyl sulfoxide |

### DESCRIPTION OF THE DRAWINGS

Figure 1 shows an X-ray powder diffraction pattern of compound ATV-014 free base crystalline form I.
Figure 2 shows an X-ray powder diffraction pattern of compound ATV-014 hydrochloride crystalline form I.
Figure 3 shows an X-ray powder diffraction pattern of compound ATV-014 hydrochloride crystalline form II.
Figure 4 shows an X-ray powder diffraction pattern of compound ATV-014 hydrochloride crystalline form III.
Figure 5 shows an X-ray powder diffraction pattern of compound ATV-014 hydrobromide crystalline form I.
Figure 6 shows an X-ray powder diffraction pattern of compound ATV-014 *p*-toluenesulfonate crystalline form I.
Figure 7 shows an X-ray powder diffraction pattern of compound ATV-014 hydrochloride crystalline form IV
Figure 8 shows a differential scanning calorimetry detection spectrum and a thermogravimetric analysis spectrum of compound ATV-014 free base crystalline form I.
Figure 9 shows a differential scanning calorimetry detection spectrum and a thermogravimetric analysis spectrum of compound ATV-014 hydrochloride crystalline form I.
Figure 10 shows a differential scanning calorimetry detection spectrum and a thermogravimetric analysis spectrum of compound ATV-014 hydrochloride crystalline form II.
Figure 11 shows a differential scanning calorimetry detection spectrum and a thermogravimetric analysis spectrum of compound ATV-014 hydrochloride crystalline form III.
Figure 12 shows a differential scanning calorimetry detection spectrum and a thermogravimetric analysis spectrum of compound ATV-014 hydrobromide crystalline form I.
Figure 13 shows a differential scanning calorimetry detection spectrum and a thermogravimetric analysis spectrum of compound ATV-014 *p*-toluenesulfonate crystalline form I.
Figure 14 shows a differential scanning calorimetry detection spectrum and a thermogravimetric analysis spectrum of compound ATV-014 hydrochloride crystalline form IV

### DETAILED DESCRIPTION

In order to make those skilled in the art better understand the technical solutions of the present invention, non limiting embodiments will be further disclosed to further describe the present invention.
The reagents used in the invention are either commercially available or can be prepared by the methods described herein.

### Example 1 Preparation of compound ATV-014 hydrochloride crystalline form I

25 mg of ATV-014 free base crystalline form I was weighed, 1 mL of THF was added to prepare a saturated solution, 1.0 equivalent of hydrochloric acid was added to the above solution for salt formation, then 1 mL of MTBE was added as an anti solvent to precipitate the solid. The resulting suspension was stirred at room temperature for 20 hours, the solid was collected through filtration and dried under vacuum at 50°C for 3 hours to obtain ATV-014 hydrochloride crystalline form I. The obtained ATV-014 hydrochloride crystalline form I was detected by X-ray powder diffraction, DSC, and TGA. The X-ray powder diffraction spectrum was shown in Figure 2, and the DSC and TGA spectra were shown in Figure 9.

### Example 2 Preparation of compound ATV-014 hydrochloride crystalline form II

0.16 mL of concentrated hydrochloric acid was diluted with IPA to 3 mL. 94 mg of ATV-014 free base crystalline form I was added into a 1.02mL of HCl IPA solution (1.05 equivalents), the mixture was stirred at room temperature for about ten minutes until the mixture becomes viscous, then 0.5mL of IPA was added, the suspension was stirred at room temperature for 2 hours, 1 mL of MTBE was added, and continued to stir for 2 hours, filtered to collect the solid. The solid washed with 3 mL of MTBE and then dried under vacuum at 50 °C for 3 hours to obtain ATV-014 hydrochloride crystalline form II. The obtained ATV-014 hydrochloride crystalline form II was detected by X-ray powder diffraction, DSC, and TGA. The X-ray powder diffraction spectrum was shown in Figure 3, and the DSC and TGA spectra were shown in Figure 10.

### Example 3 Preparation of compound ATV-014 hydrochloride crystalline form III

69 mg of ATV-014 free base crystalline form I was suspended in 1 mL of dichloromethane, and stirred at room temperature for about 10 minutes, then 1.0 equivalent of concentrated hydrochloric acid was added and stirred for 2 hours, the solid was filtered and dried under vacuum at 50 °C for 3 hours to obtain ATV-014 hydrochloride crystalline form III. The obtained ATV-014 hydrochloride crystalline form III was detected by X-ray powder diffraction, DSC, and TGA. The X-ray powder diffraction spectrum was shown in Figure 4, and the DSC and TGA spectra were shown in Figure 11.

### Example 4 Preparation of compound ATV-014 hydrobromide crystalline form I

Approximately 500 mg of free base crystalline form I was suspended in 15 mL of ethyl acetate, and the resulting suspension was stirred at room temperature for 10 minutes. Then, 1 equivalent of hydrobromic acid was added and stirred at room temperature for 2 hours. The solid was filtered and collected, then dried overnight under vacuum at 50°C to obtain ATV-014 hydrobromate crystalline form I. The obtained ATV-014 hydrobromate crystalline form I was detected by X-ray powder diffraction, DSC, and TGA. The X-ray powder diffraction spectrum was shown in Figure 5, and the DSC and TGA spectra were shown in Figure 12.

### Example 5 Preparation of compound ATV-014 p-toluenesulfonate crystalline form I

Approximately 500 mg of free base crystalline form I was suspended in 15 mL of isopropanol, and then 1 equivalent of solid *p*-toluenesulfonic acid was added, most of the solid was dissolved. The suspension was stirred at room temperature for 2 hours without any further solid precipitation. The crystalline seeds of *p*-toluenesulfonate was added, then continued to stirred for 2 hours, filtered to collect the solid. The obtained solid was dried overnight at room temperature, and then dried under vacuum at 40°C for 2 hours to obtain ATV-014 *p*-toluenesulfonate crystalline form I. The obtained ATV-014 *p*-toluenesulfonate crystalline form I was detected by X-ray powder diffraction, DSC, and TGA. The X-ray powder diffraction spectrum was shown in Figure 6, and the DSC and TGA spectra were shown in Figure 13.

### Example 6 Preparation of compound ATV-014 hydrochloride crystalline form IV

ATV014 (1 g, 2.5 mmol) and 5 mL of methanol (almost insoluble) were added into a 50 mL single necked flask. A 4.0 M hydrochloric acid solution in methanol (0.9 mL, 3.75 mmol) was added dropwise under ice bath conditions. After 2 minutes, the solution becomes clear and transparent. 20 mL of MTBE directly was added to the residue, and stirred at room temperature for 2 hours, and a large amount of white solid was formed, filtered and dried under vacuum at 45 ° C to obtain 1 g of ATV014 hydrochloride. The obtained ATV-014 hydrochloride crystalline form IV was detected by X-ray powder diffraction, DSC, and TGA. The X-ray powder diffraction spectrum was shown in Figure 7, and the DSC and TGA spectra were shown in Figure 14.

### Example 7 Preparation of compound ATV-014 free base crystalline form I

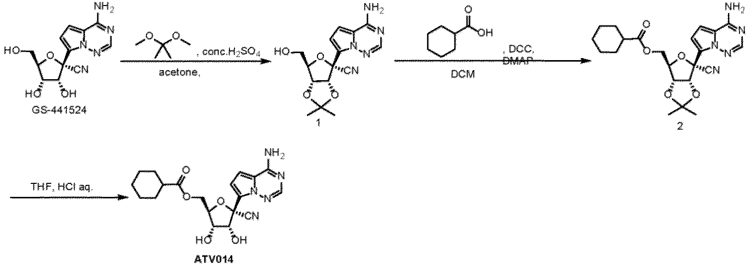

GS-441524 (10 g, 0.03 mol) was added into a 500 mL reactor equiped with a stirrer, thermometer, and constant pressure drip funnel, acetone dried over magnesium sulfate (300 mL) was added, then 2,2-dimethoxypropane (17 g, 0.16 mol) was added, and concentrated sulfuric acid (2.4 mL, 0.04 mol) was added dropwise to the system at room temperature. After 5 minutes, the dropwise addition was complete, and the solid began to dissolve. Heating up to 45 ° C and the reaction was continued for 4 hours, the reaction was monitored by HPLC(OD-3 column, mobile phase: n-hexane/isopropanol=80:20, flow rate: 0.8 mL/min, injection amount 1 µ L) until complete, the reaction was stopped, and cooled in an ice bath, solid NaHCOs (10 g) and water (30 mL) were added to the reaction solution, the mixture pH was adjusted to 7-8 with sodium bicarbonate, the solvent was removed through vacuum distillation, the remaining substance was diluted with ethyl acetate (300 mL), ethyl acetate layers were washed with water (80 mL) and saturated sodium chloride aqueous solution (80 mL), and dried over anhydrous sodium sulfate. Filtering, the filtrate was distiled under reduced pressure to about 100 mL. The residue was slowly poured into petroleum ether cooled in an ice bath and stirred vigorously to precipitate out a large amount of white solid. Compound 1 was obtained by filtration with a yield of 91%, 10.5 g of white solid.

15.0 g of Compound 1 was dissolved in 15 mL of dichloromethane, then cyclohexanecarboxylic acid and 554.0 mg of 4-dimethylaminopyridine were added thereto, and after stirring for 10 min, 10.2 g of dicyclohexylcarbodiimide was added, and the mixture was stirred at room temperature for 24 hours. The mixture was purified by column chromatography (eluent: petroleum ether/ethyl acetate (V/V) = 1/1) to obtain of compound 2 (white solid yield). Compound 2 was dissolved in 30 mL of hydrochloric acid aqueous solution with 37% mass percentage and 150 mL of tetrahydrofuran, the mixture was stirred for 6 hours, sodium carbonate was added to adjust pH to 8, the organic solvents were removed by rotary evaporation, and separated by column chromatography (elution solution: petroleum ether/ethyl acetate (V/V)=1/3) to obtain 2.8 g compound ATV014 (free base crystalline form I, yield 49%), with a two-step yield of 45.8%. The obtained ATV-014 free base crystalline form I was detected by X-ray powder diffraction, DSC, and TGA. The X-ray powder diffraction spectrum was shown in Figure 1, and the DSC and TGA spectra were shown in Figure 8. The obtained compound E1TV014 was tested for hydrogen spectrum, and the results were as follows:
Hydrogen spectrum: ¹H NMR (600 MHz, DMSO-*d*₆) δ (ppm): 7.92 (s, 1H), 7.86 (br, 1H), 6.92 (d, *J*=4.5 Hz,1H), 6.81 (d, *J*=4.5 Hz, 1H), 6.33 (d, *J*=5.9 Hz, 1H), 5.38 (d, *J*=5.9 Hz, 1H), 4.70 (t, *J*=5.3 Hz, 1H), 4.32-4.29 (dd, *J*=12.2 Hz, 2.6 Hz, 1H), 4.24-4.21 (m, 1H), 4.16-4.13 (dd, *J*=12.3 Hz, 4.8 Hz, 1H), 3.98-3.95 (q, J=5.9 Hz, 1H), 2.26-2.22 (m, 1H), 1.75-1.72 (m, 2H), 1.64-1.56 (m, 3H), 1.30-1.12 (m, 5H).
Carbon spectrum: ¹³C NMR (150 MHz, DMSO-*d*₆) δ (ppm): 175.34, 156.06, 148.4, 124.0, 117.4, 117.0, 110.7, 101.2, 81.7, 79.4, 74.5, 70.6, 63.0, 42.6, 29.0, 28.9, 25.7, 25.2, 25.1.

### Example 8 Study on the Stability of Influence Factors

ATV-014 free base crystalline form I, ATV-014 hydrobromate crystalline form I, and ATV-014 *p*-toluenesulfonate crystalline form I were taken and placed at 40 °C/75% RH (open) and 60 °C (sealed sample bottle) for 7 days. High-performance liquid chromatography and X-ray powder diffraction detection were performed at 0 and 7 days, respectively. The results were shown in Table 4.

**Table 4 Study on the Stability of Influence Factors**

| **Sample** | **Purity at day 0 (Peak area%)** | **Purity at day 7 (Peak area%)** | |
|---|---|---|---|
| | | **60 °C** | **40 °C/75%RH** |
| ATV-014 free base crystalline form I | 98.12% | 98.10% | 98.09% |
| ATV-014 hydrobromide crystalline form I | 99.46% | 99.57% | Liquefaction, undetected |
| ATV-014 *p*-toluenesulfonate crystalline form I | 98.60% | 98.62% | 98.71% |

Conclusion: The results showed that under experimental conditions (60°C and 40°C/75% RH) for 7 days, the purity and crystalline structure of ATV-014 free base crystalline form I and ATV-014 *p*-toluenesulfonate crystalline form I remained unchanged, and their physical and chemical stability were good; ATV-014 hydrobromate crystalline form I remained unchanged in purity and crystalline form after being placed under experimental conditions (60°C) for 7 days; After being placed at 40°C/75% RH for 7 days, the sample liquefies.

### Example 9: Solubility study

According to the results of salt formation screening research, the solubilities of free base crystalline form I and *p*-toluenesulfonate crystalline form I at 0.5 hours, 2 hours, and 24 hours were measured in FaSSIF, FeSSIF, SGF, and water at 37 °C. The results of solubility and solution pH were shown in Tabletable 5.

**Table 5. Solubility results in biological media**

| **Sample** | **media** | **0.5 h** | | | **2h** | | **24 h** | | |
|---|---|---|---|---|---|---|---|---|---|
| | | **solubility (mg/mL)** | **XPRD** | **pH** | **solubility (mg/mL)** | **pH** | **solubility (mg/mL)** | **XPRD** | **pH** |
| free base crystallin e form I | FaSSIF, pH 6.45 | 0.007 | / | 6.45 | 0.01 | 6.44 | 0.021 | No changed | 6.58 |
| | FeSSIF, pH 4.95 | 0.014 | / | 4.76 | 0.019 | 4.74 | 0.020 | No changed | 4.96 |
| | SGF, pH 1.24 | 2.48 | / | 0.95 | 2.56 | 0.9 | 2.86 | No changed | 1.26 |
| | water, pH 5.53 | 0.005 | / | 7.24 | 0.006 | 6.45 | 0.005 | No changed | 6.5 |
| *p*-toluene sulfonate crystallin e form I | FaSSIF, pH 6.45 | 0.010 | free base crystalline form I | 5.7 | 0.010 | 5.67 | 0.008 | free base crystalline form I | 5.85 |
| | FeSSIF, pH 4.95 | 0.020 | free base crystalline form I | 4.65 | 0.016 | 4.62 | 0.018 | free base crystalline form I | 4.83 |
| | SGF, pH 1.24 | >5 | / | 0.69 | >5 | 0.70 | >5 | / | 1.20 |
| | water, pH 5.53 | 0.15 | free base crystalline form I | 2.05 | 0.144 | 2.07 | 0.156 | free base crystalline form I | 2.43 |

The results showed that the solubility of the sample has pH dependence, with high solubility under low pH conditions, and the higher the solubility of the sample. The solubility of ATV-014 *p*-toluenesulfonate crystalline form I is higher than 5 mg/mL, and the solubility of ATV-014 free base crystalline form I in SGF is 2.5-2.9 mg/mL. In addition, the solubility of ATV-014 *p*-toluenesulfonate in water is 30 times higher than that of free base.

The method of the present invention has been described through preferred embodiments, and it is evident that relevant personnel can modify or appropriately modify and combine the methods and applications described herein within the content, spirit, and scope of the present invention to achieve and apply the technology of the present invention. Technicians in this field can refer to the content of this article and appropriately improve the implementation of process parameters. It should be noted that all similar replacements and modifications are apparent to those skilled in the art and are considered to be included in the present invention.

## Claims

1. Compound ATV014 hydrochloride crystalline form I: 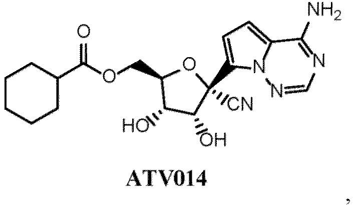 which is **characterized by** an X-ray powder diffraction pattern of compound ATV014 hydrochloride crystalline form I having characteristic peaks at 2Θ ± 0.2° values of 5.16, 15.53 and 20.76; or an X-ray powder diffraction pattern of compound ATV014 hydrochloride crystalline form I having characteristic peaks at 2Θ ± 0.2° values of 5.16, 10.33, 15.53, 20.76, 22.75 and 24.19; or an X-ray powder diffraction pattern of compound ATV014 hydrochloride crystalline form I having characteristic peaks at 2Θ ± 0.2° values of 5.16, 10.33, 15.53, 20.76, 22.75, 23.88, 24.19 and 26.01.

2. The compound ATV014 hydrochloride crystalline form I of claim 1, wherein the X-ray powder diffraction pattern of compound ATV014 hydrochloride crystalline form I has characteristic peaks at 2Θ ± 0.2° values of 5.16, 15.53 and 20.76, and further has one, two or three characteristic peaks at 2Θ ± 0.2° values of 10.33, 22.75 and 24.19; or the X-ray powder diffraction pattern of compound ATV014 hydrochloride crystalline form I has characteristic peaks at 2Θ ± 0.2° values of 5.16, 10.33, 15.53, 20.76, 22.75 and 24.19, and further has one or two characteristic peaks at 2Θ ± 0.2° values of 23.88 and 26.01.

3. The compound ATV014 hydrochloride crystalline form I of claim 1, wherein the X-ray powder diffraction pattern of compound ATV014 hydrochloride crystalline form I is substantially as depicted in Fig.2; and/or the differential scanning calorimetry curve of compound ATV014 hydrochloride crystalline form I has an endothermic peak with a peak temperature of 120 °C - 170 °C; or the differential scanning calorimetry curve of compound ATV014 hydrochloride crystalline form I has an endothermic peak with a peak temperature of 125 °C - 165 °C; or the differential scanning calorimetry curve of compound ATV014 hydrochloride crystalline form I has an endothermic peak with a peak temperature of 141 °C - 151 °C or 143 °C - 149 °C; and/or
the thermogravimetric analysis curve of compound E1TV014 hydrochloride crystalline form I has a weight loss of less than 10% or less than 9% or less than 8% in a temperature range of 110 °C to170 °C; the thermogravimetric analysis curve of compound ATV014 hydrochloride crystalline form I is substantially as depicted in Fig.9.

4. Compound ATV014 hydrochloride crystalline form II, 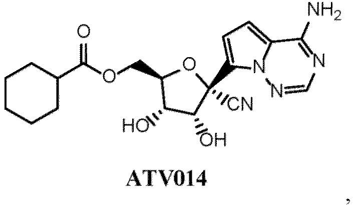 which is **characterized by** an X-ray powder diffraction pattern of compound ATV014 hydrochloride crystalline form II having characteristic peaks at 2Θ ± 0.2° values of 5.23, 10.43 and 18.79; or an X-ray powder diffraction pattern of compound ATV014 hydrochloride crystalline form II having characteristic peaks at 2Θ ± 0.2° values of 5.23, 9.88, 10.43, 14.05 and 18.79 and 22.28; or an X-ray powder diffraction pattern of compound ATV014 hydrochloride crystalline form II having characteristic peaks at 2Θ ± 0.2° values of 5.23, 9.88, 10.43, 14.05, 15.68, 18.79, 19.46, 21.38 and 22.28; or an X-ray powder diffraction pattern of compound ATV014 hydrochloride crystalline form II having characteristic peaks at 2Θ ± 0.2° values of 5.23, 9.88, 10.43, 14.05, 15.68, 17.81, 18.79, 19.46, 20.37, 21.38, 22.28 and 23.96; or an X-ray powder diffraction pattern of compound ATV014 hydrochloride crystalline form II having characteristic peaks at 2Θ ± 0.2° values of 5.23, 9.88, 10.43, 14.05, 15.68, 17.81, 18.79, 19.46, 20.37, 21.38, 22.28, 22.81, 23.96 and 37.05.

5. The compound ATV014 hydrochloride crystalline form II of claim 4, wherein the X-ray powder diffraction pattern of compound ATV014 hydrochloride crystalline form II has characteristic peaks at 2Θ ± 0.2° values of 5.23, 10.43 and 18.79, and further has one, two or three characteristic peaks at 2Θ ± 0.2° values of 9.88, 14.05 and 22.28; or the X-ray powder diffraction pattern of compound ATV014 hydrochloride crystalline form II has characteristic peaks at 2Θ ± 0.2° values of 5.23, 9.88, 10.43, 14.05, 18.79 and 22.28, and further has one, two or three characteristic peaks at 2Θ ± 0.2° values of 15.68, 19.46 and 21.38; or the X-ray powder diffraction pattern of compound ATV014 hydrochloride crystalline form II has characteristic peaks at 2Θ ± 0.2° values of 5.23, 9.88, 10.43, 14.05, 15.68, 18.79, 19.46, 21.38 and 22.28, and further has one, two or three characteristic peaks at 2Θ ± 0.2° values of 17.81, 20.37 and 23.96; or the X-ray powder diffraction pattern of compound ATV014 hydrochloride crystalline form II has characteristic peaks at 2Θ ± 0.2° values of 5.23, 9.88, 10.43, 14.05, 15.68, 17.81, 18.79, 19.46, 20.37, 21.38, 22.28 and 23.96, and further has one or two characteristic peaks at 2Θ ± 0.2° values of 22.81 and 37.05.

6. The compound ATV014 hydrochloride crystalline form II of claim 4, wherein the X-ray powder diffraction pattern of compound ATV014 hydrochloride crystalline form II is substantially as depicted in Fig.3; and/or
the differential scanning calorimetry curve of compound ATV014 hydrochloride crystalline form II has an endothermic peak with a peak temperature of 100°C - 150°C; or the differential scanning calorimetry curve of compound ATV014 hydrochloride crystalline form II has an endothermic peak with a peak temperature of 135 °C - 145 °C or 136 °C - 143 °C; or the differential scanning calorimetry curve of compound ATV014 hydrochloride crystalline form II is substantially as depicted in Fig. 10; and/or the thermogravimetric analysis curve of compound ATV014 hydrochloride crystalline form II has a weight loss of less than 10% or less than 8% in a temperature range of 70 °C to 170 °C;
or the thermogravimetric analysis curve of compound ATV014 hydrochloride crystalline form II is substantially as depicted in Fig.10.

7. Compound ATV014 hydrochloride crystalline form III, 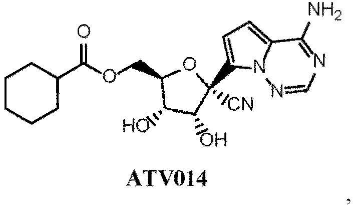 which is **characterized by** an X-ray powder diffraction pattern of compound ATV014 hydrochloride crystalline form III having characteristic peaks at 2Θ ± 0.2° values of 5.28, 14.03 and 18.99; or an X-ray powder diffraction pattern of compound ATV014 hydrochloride crystalline form III having characteristic peaks at 2Θ ± 0.2° values of 5.28, 10.54, 14.03, 15.84, 18.99 and 21.16; or an X-ray powder diffraction pattern of compound ATV014 hydrochloride crystalline form III having characteristic peaks at 2Θ ± 0.2° values of 5.28, 10.54, 14.03, 15.84, 17.67, 18.99, 21.16, 22.49 and 27.51.

8. The compound ATV014 hydrochloride crystalline form III of claim 7, wherein the X-ray powder diffraction pattern of compound ATV014 hydrochloride crystalline form III has characteristic peaks at 2Θ ± 0.2° values of 5.28, 14.03 and 18.99, and further has one, two or three characteristic peaks at 2Θ ± 0.2° values of 10.54, 15.84 and 21.16; or the X-ray powder diffraction pattern of compound ATV014 hydrochloride crystalline form III has characteristic peaks at 2Θ ± 0.2° values of 5.28, 10.54, 14.03, 15.84, 18.99 and 21.16, and further has one, two or three characteristic peaks at 2Θ ± 0.2° values of 17.67, 22.49 and 27.51.

9. The compound ATV014 hydrochloride crystalline form III of claim 4, wherein the X-ray powder diffraction pattern of compound ATV014 hydrochloride crystalline form III is substantially as depicted in Fig.4; and/or
the differential scanning calorimetry curve of compound ATV014 hydrochloride crystalline form III has an endothermic peak with a peak temperature of 90 °C - 170 °C; or the differential scanning calorimetry curve of compound ATV014 hydrochloride crystalline form III has an endothermic peak with a peak temperature of 125 °C - 170 °C; or the differential scanning calorimetry curve of compound ATV014 hydrochloride crystalline form III has an endothermic peak with peak temperature of 130 °C - 138 °C; or the differential scanning calorimetry curve of compound ATV014 hydrochloride crystalline form III is substantially as depicted in Fig.11; and/or
the thermogravimetric analysis curve of compound ATV014 hydrochloride crystalline form III has a weight loss of less than 10% or less than 9% in a temperature range of 70 °C to170 °C;
the thermogravimetric analysis curve of compound ATV014 hydrochloride crystalline form III is substantially as depicted in Fig.11.

10. Compound ATV014 hydrochloride crystalline form IV, 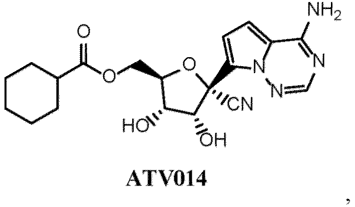 which is **characterized by** an X-ray powder diffraction pattern of compound ATV014 hydrochloride crystalline form IV having characteristic peaks at 2Θ ± 0.2° values of 5.28, 10.63 and 19.13; or an X-ray powder diffraction pattern of compound ATV014 hydrochloride crystalline form IV having characteristic peaks at 2Θ ± 0.2° values of 5.28, 9.63, 10.63, 14.10, 16.82 and 19.13; or an X-ray powder diffraction pattern of compound ATV014 hydrochloride crystalline form IV having characteristic peaks at 2Θ ± 0.2° values of 5.28, 9.63, 10.19, 10.63, 14.10, 15.99, 16.82, 17.62 and 19.13; or an X-ray powder diffraction pattern of compound ATV014 hydrochloride crystalline form IV having characteristic peaks at 2Θ ± 0.2° values of 5.28, 9.63, 10.19, 10.63, 14.10, 15.99, 16.82, 17.62, 19.13, 22.27, 23.19 and 27.74.

11. The compound ATV014 hydrochloride crystalline form IV of claim 10, wherein the X-ray powder diffraction pattern of compound ATV014 hydrochloride crystalline form IV has characteristic peaks at 2Θ ± 0.2° values of 5.28, 10.63 and 19.13, and further has one, two or three characteristic peaks at 2Θ ± 0.2° values of 9.63, 14.10 and 16.82; or the X-ray powder diffraction pattern of compound ATV014 hydrochloride crystalline form IV has characteristic peaks at 2Θ ± 0.2° values of 5.28, 9.63, 10.63, 14.10, 16.82 and 19.13, and further has one, two or three characteristic peaks at 2Θ ± 0.2° values of 10.19, 15.99 and 17.62; or the X-ray powder diffraction pattern of compound ATV014 hydrochloride crystalline form IV has characteristic peaks at 2Θ ± 0.2° values of 5.28, 9.63, 10.19, 10.63, 14.10, 15.99, 16.82, 17.62 and 19.13, and further has one, two or three characteristic peaks at 2Θ ± 0.2° values of 22.27, 23.19 and 27.74.

12. The compound ATV014 hydrochloride crystalline form IV of claim 10, wherein the X-ray powder diffraction pattern of compound ATV014 hydrochloride crystalline form IV is substantially as depicted in Fig.5; and/or
the differential scanning calorimetry curve of compound ATV014 hydrochloride crystalline form IV has an endothermic peak with a peak temperature of 180 °C - 200 °C; or the differential scanning calorimetry curve of compound ATV014 hydrochloride crystalline form IV has an endothermic peak with peak temperature of 183 °C - 190 °C; or the differential scanning calorimetry curve of compound ATV014 hydrochloride crystalline form IV is substantially as depicted in Fig.12; and/or
the thermogravimetric analysis curve of compound ATV014 hydrochloride crystalline form IV has a weight loss of less than 2% or less than 1% or less than 0.5% or less than 0.2% in a temperature range of 30 °C to 150 °C;
or the thermogravimetric analysis curve of compound ATV014 hydrochloride crystalline form IV is substantially as depicted in Fig.12.

13. Compound ATV014 hydrobromate crystalline form I, 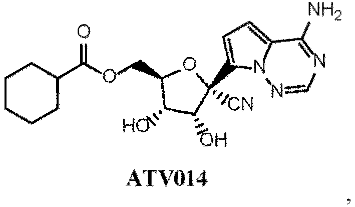 which is **characterized by** an X-ray powder diffraction pattern of compound ATV014 hydrobromate crystalline form I having characteristic peaks at 2Θ ± 0.2° values of 3.78, 13.20 and 16.88; or an X-ray powder diffraction pattern of compound ATV014 hydrobromate crystalline form I having characteristic peaks at 2Θ ± 0.2° values of 3.78, 13.20, 15.41, 16.88, 17.46 and 26.38; or an X-ray powder diffraction pattern of compound ATV014 hydrobromate crystalline form I having characteristic peaks at 2Θ ± 0.2° values of 3.78, 13.20, 15.41, 16.88, 17.46, 25.52, 26.38 and 26.79.

14. The compound ATV014 hydrobromate crystalline form I of claim 13, wherein the X-ray powder diffraction pattern of compound ATV014 hydrobromate crystalline form I has characteristic peaks at 2Θ ± 0.2° values of 3.78, 13.20 and 16.88, and further has one, two or three characteristic peaks at 2Θ ± 0.2° values of 15.41, 17.46 and 26.38; or the X-ray powder diffraction pattern of compound ATV014 hydrobromate crystalline form I has characteristic peaks at 2Θ ± 0.2° values of 3.78, 13.20, 15.41, 16.88, 17.46 and 26.38, and further has one or two characteristic peaks at 2Θ ± 0.2° values of 25.52 and 26.79.

15. The compound ATV014 hydrobromate crystalline form I of claim 13, wherein the X-ray powder diffraction pattern of compound ATV014 hydrobromate crystalline form I is substantially as depicted in Fig.7; and/or
the differential scanning calorimetry curve of compound ATV014 hydrobromate crystalline form I has an endothermic peak with a peak temperature of 130 °C - 170 °C; or the differential scanning calorimetry curve of compound ATV014 hydrobromate crystalline form I has an endothermic peak with peak temperature of 138 °C - 145 °C; or the differential scanning calorimetry curve of compound ATV014 hydrobromate crystalline form I is substantially as depicted in Fig.14; and/or
the thermogravimetric analysis curve of compound E1TV014 hydrobromate crystalline form I has a weight loss of less than 2% or less than 1.5% in a temperature range of 100 °C to 160 °C; or the thermogravimetric analysis curve of compound ATV014 hydrobromate crystalline form I is substantially as depicted in Fig.14.

16. Compound ATV014 *p*-toluenesulfonate crystalline form I, 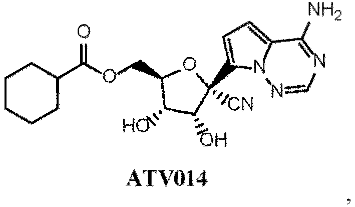 which is **characterized by** an X-ray powder diffraction pattern of compound ATV014 *p*-toluenesulfonate crystalline form I having characteristic peaks at 2Θ ± 0.2° values of 3.58, 10.89 and 16.59; or an X-ray powder diffraction pattern of compound ATV014 *p*-toluenesulfonate crystalline form I having characteristic peaks at 2Θ ± 0.2° values of 3.58, 7.23, 10.89, 14.59 and 16.59.

17. The compound ATV014 *p*-toluenesulfonate crystalline form I of claim 16, wherein the X-ray powder diffraction pattern of compound ATV014 *p*-toluenesulfonate crystalline form I is substantially as depicted in Fig.6; and/or
the differential scanning calorimetry curve of compound ATV014 *p*-toluenesulfonate crystalline form I has an endothermic peak with a peak temperature of 100 °C - 140°C; or the differential scanning calorimetry curve of compound ATV014 *p*-toluenesulfonate crystalline form I has an endothermic peak with peak temperature of 120 °C - 127 °C; or the differential scanning calorimetry curve of compound ATV014 *p*-toluenesulfonate crystalline form I is substantially as depicted in Fig. 13; and/or
the thermogravimetric analysis curve of compound ATV014 *p*-toluenesulfonate crystalline form I has a weight loss of less than 10% or less than 5% or less than 4% in a temperature range of 60 °C to 150 °C; and/or the thermogravimetric analysis curve of compound ATV014 *p*-toluenesulfonate crystalline form I is substantially as depicted in Fig. 13.

18. A pharmaceutical composition comprising a therapeutically effective amount of compound ATV014 hydrochloride crystalline form I of any one of claims 1 to 3, a therapeutically effective amount of compound ATV014 hydrochloride crystalline form II of any one of claims 4 to 6, a therapeutically effective amount of compound ATV014 hydrochloride crystalline form III of any one of claims 7 to 9, a therapeutically effective amount of compound ATV014 hydrochloride crystalline form IV of any one of claims 10 to 12, a therapeutically effective amount of compound ATV014 hydrobromate crystalline form I of any one of claims 13 to 15 or a therapeutically effective amount of compound ATV014 *p*-toluenesulfonate crystalline form I of any one of claims 16 to 17, and optionally a pharmaceutically acceptable excipient.

19. Use of compound ATV014 hydrochloride crystalline form I of any one of claims 1 to 3, compound ATV014 hydrochloride crystalline form II of any one of claims 4 to 6, compound ATV014 hydrochloride crystalline form III of any one of claims 7 to 9, compound ATV014 hydrochloride crystalline form IV of any one of claims 10 to 12, compound ATV014 hydrobromate crystalline form I of any one of claims 13 to 15 or compound ATV014 *p*-toluenesulfonate crystalline form I of any one of claims 16 to 17, or pharmaceutical composition of claim 18 in the manufacture of a medicament for preventing, lessening or treating a disease or symptom related to viral infections.
